# EUROPEAN PATENT APPLICATION

(11) **EP 4 455 272 A1**
(43) Date of publication of application: **30.10.2024**
(21) Application number: 23170510.4
(22) Date of filing: 27.04.2023
(51) Int. Cl.: C12N 5/071

(54) **METHOD FOR PRODUCING MILK LIKE PRODUCTS**

(71) Applicant: Société des Produits Nestlé S.A., 1800 Vevey (CH)
(72) Inventor:
(74) Representative: CMS Cameron McKenna Nabarro Olswang LLP

(57) **Abstract**

The invention provides isolated human breast milk exosomes, and compositions thereof. The present invention also provides *in vitro* methods for producing said exosomes comprising generating lactocytes derived from human induced pluripotent stem cells (hiPSC), expressing the human milk like product from lactocytes, and purifying the exosomes from the human milk like product.

## Description

### Field of the invention

The present invention concerns isolated human breast milk exosomes, and compositions thereof. The present invention also relates to *in vitro* methods for producing said exosomes, the method comprising generating lactocytes derived from human induced pluripotent stem cells (hiPSC), through culture and differentiation, and/or mammary-like gland organoids comprising such lactocytes, expressing a human milk like product from such lactocytes and/or mammary-like gland organoids, and purifying the exosomes from the human milk like product.

### Background of the invention

Mammalian and especially human milk is a complex fluid with a multitude of components, each of which may contribute substantially to infant and perhaps maternal health. It is becoming increasingly clear that human breastmilk is the most appropriate source of nutrition at least up to the age of 6 months. Many components of human milk are simply not found or poorly found or less active in cow's milk upon which infant formula manufacture is based. This includes for instance protein lactoferrin, growth factors, long chain polyunsaturated fatty acids or oligosaccharides. Human milk composition has been used as a gold standard to develop current infant formula, despite recent major development in infant formula composition, it is illusory to think that human milk replication or replication of components of said human milk will be achieved with current manufacturing processes.

Today the only source of human milk is human donors (breastfeeding mothers). Donation are reported for non-commercial use (human milk biobank) and commercial use. However, this is limited and has strong regulatory, safety and sometime ethical or religious constraints.

Stem cells were found in mammalian and especially human milk called human breastmilk stem cells (hBSC). hBSCs were shown to be highly plastic and to differentiate in culture into multiple cell types and more importantly into the three lineages required to shape the lobulo-alveolar structure of the human mammary gland (Hassiotou F. et al. Stem Cells. 2012). However, the use of hBSC to produce human breast milk is neither practical nor sustainable, as it requires human donors.

A technology based on a cell line with stem cell functionality - called induced pluripotent stem cells (iPSC) is known. A reliable two step protocol to generate human mammary like organoids from human iPSC (hiPSC) was developed (Ying Qu et al, Stem Cell Report vol 8, 205-215, February 14th 2017).

Accordingly, it is an object of the present invention to provide improved methods for producing human milk and key components of human milk such as exosomes using cultured cells. It is also an object of the present invention to prepare customized human milk like product, in cultured cells which could be adapted to specific needs of the recipient and/or to produce human milk bioactives such as exosomes to complement existing cow-based solutions for infant nutrition.

### Summary of the invention

The present invention solves the above-mentioned technical problem.

Provided herein is an isolated human breast milk exosome, wherein the exosome does not comprise one or more proteins selected from C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine-tRNA ligase 1, cytoplasmic (TARS1), and Cytosolic non-specific dipeptidase (CNDP2).

Also provided herein is a population of exosomes, wherein the population comprises exosomes as described anywhere herein.

Also provided herein is a composition comprising exosomes or a population of exosomes as described anywhere herein.

Also provided herein is an *in vitro* method for producing human breast milk exosomes, the method comprising:
A) Generating lactocyte mammary-like gland organoids derived from human induced pluripotent stem cells (hiPSC)
B) Secreting a human milk like product from said lactocytes, and
C) Purifying the exosomes from the human milk like product to remove impurities, optionally by chromatography or filtration or ultracentrifugation, in order to isolate the exosomes,
wherein step A) comprises culturing the hiPSCs in a culture medium comprising BMP4 and/or RA.

Also provided herein is a human breast milk exosome product which is obtainable according to the method as described anywhere herein.

Finally, provided herein is a method of producing a human milk fortifier, comprising conducting the method as described anywhere herein.

### Detailed description of the invention

### Definitions

Within the context of the present invention, the term *"in vitro"* means performed or taking place in a test tube, culture dish, bioreactor or elsewhere outside a living organism.

Within the context of the present invention, the term "mammalian" identify an animal belonging to the mammalian species, for example human, cow, monkey, camel, sheep, goat etc.

Within the context of the present invention, the terms "lactocytes" or "mammary-like cells" identify secretory epithelial cells expressing CK18 cell marker and derived from mammalian induced pluripotent stem cells (miPSC) and in particular from human induced pluripotent stem cells (hiPSC). Human induced pluripotent stem cells (hiPSC) as used herein are commercially available and may be selected from any suitable hiPSC line. A suitable human induced pluripotent stem cell line in the context of the current invention is e.g., hiPSC line 603, commercially available from Fujifilm Cellular Dynamics, Inc (FCDI), as used according to the invention. Further suitable hiPSCs may be selected as described e.g., in Ying Qu et al., (2017, supra). In one embodiment of the present invention, the hiPSC are not engineered. In one embodiment, they are not engineered to comprise an exogenous nucleic acid and/or an inducible gene expression system which includes an exogenous nucleic acid, where the inducible gene expression system is configured to express a hormone or a signaling factor. In one embodiment, the exogenous nucleic acid and/or inducible gene expression system which includes an exogenous nucleic acid is promoting the cell differentiation towards lactocytes.

Within the context of the present invention the terms "mammary gland like organoids" or "mammary like organoids" mean a miniaturized and simplified version of a mammary gland which develops in two or three dimensions (2D/3D) and which comprises lactocytes as above defined.

Within the context of the present invention, the term "human milk like product" is a cell cultured milk product. It is an edible product which is expressed by the lactocytes and/or mammary gland like organoids generated according to the process of the present invention.

The "human milk like product" according to the present invention can have the same components as human breast milk of a well-nourished mother (for example in terms of bioactives, macro and micronutrients and levels thereof). This is referred to herein as a "standard human milk product". Alternatively, "human milk like product" according to the present invention can have altered ratios and concentrations of components found naturally in human breast milk of a well-nourished mother. This is referred to herein as a "non-standard milk like product". A "human milk like product" according to the present invention can be modified such that it includes components that are not found naturally in human breast milk of a well-nourished mother (a "modified milk like product"). Non-limiting examples of human milk like products are selected from the group consisting of: supplement, fortifier, human breast milk substitute (or replacer) and ingredient enriched in only one and/or a portion of bioactives, macro- and micronutrients which can be typically found in human breast milk of a well-nourished mother.

The "human milk like product" can be used to replace consumption of naturally lactated milk (a "human milk substitute"). The milk substitute product can be used as a supplement (a "human milk supplement") or as a fortifier (a "human milk fortifier") to be consumed in combination with naturally lactated milk.

In an embodiment, the standard human milk like product according to the present invention comprises at least macro- and micronutrients which can be typically found in human breast milk of a well-nourished mother. In one embodiment, the human milk like product according to the present invention comprises: proteins, peptides, lipids (including linoleic acid and alpha-linolenic acid), carbohydrates, Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol and L-carnitine. In one embodiment, the human milk like product according to the present invention also comprises at least one bioactive selected from the group consisting of: growth factors, cytokines, probiotics, extracellular vesicles (e.g. milk fat globules and or exosomes), bioactives from exosome (for example miRNA) and secretory IgA.The standard human milk like product according to the present invention is not the product of human breast lactation as occurring in nature.

In another embodiment, the human milk like product according to the present invention can be adapted to specific needs of the infant who will receive it. It may comprise only one and/or a portion of bioactives, macro and micro nutrients which can be typically found in human breast milk of a well-nourished mother. In such embodiment, the human breast milk like product may also be referred to with the term "non-standard human milk like product". In one embodiment, the non-standard human milk like product according to the present invention comprises one or more of the nutrients or bioactives selected from the group consisting of proteins, peptides, lipids (including linoleic acid and alpha-linolenic acid), carbohydrates (including human milk oligosaccharides), Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol, L-carnitine, growth factors, cytokines, probiotics, extracellular vesicles (e.g. milk fat globules and or exosomes), bioactives from exosome (for example miRNA) and secretory IgA.

Within the context of the present invention, the term "non-modified human milk like product" indicates a human milk like product which is expressed by lactocytes and/or by the mammary gland like organoids generated according to steps A) and B) of the process of the present invention and which is not subject to the further treatment according to optional step C) of the process of present invention. Non-modified human milk like product may comprise both standard and non-standard human milk like products. Non limiting examples of non-standard human milk like products are selected from the group consisting of: supplement, fortifier, and ingredient enriched in only one and/or a portion of bioactives, macro and micro nutrients which can be typically found in human breast milk of a well-nourished mother.

Within the context of the present invention, the term "modified human milk like product" indicates a human milk like product which is expressed by lactocytes and/or by the mammary gland like organoids generated according to steps A) and B) of the process of the present invention and which is subject to the further treatment according to optional step C) of the process of present invention.

Modified human milk like products may comprise both standard and non-standard human milk like products.

Within the context of the present invention the term "EBs" means "embryoid bodies".

Within the context of the present invention the term "mEBs" means "MammoCult medium-cultured embryoid bodies".

MammoCult medium refers to a serum-free culture medium comprising basal medium, at least one proliferation supplement, heparin and hydrocortisone.

Within the context of the present invention the terms "embryoid bodies (EBs)", "MammoCult medium-cultured embryoid bodies (mEBs)", "mammospheres" and/or "spheroids" refer to three-dimensional aggregates formed in suspension by pluripotent stem cells (PSC) under step A) of the process of the present invention.

The term "infant" in the context of the present invention identifies a child under the age of 12 months, such as under the age of 9 months, particularly under the age of 6 months.

In the context of the present invention the infant may be any term infant or preterm infant. In an embodiment of the invention, the infant is selected from the group of preterm infants and term infants.

The term "term infant" refers to infants born at term or at a gestational age of 37 weeks or more.

The term "preterm infant" refers to infants who are born at a gestational age of less than 37 weeks.

In the context of the present invention, the term "birth weight" means the first weight of the fetus or newborn obtained after birth.

Within the context of the present invention, the term "low birth weight" means a birth weight of less than 2500 g (up to and including 2499 g).

Within the context of the present invention, the term "very low birth weight" means a birth weight of less than 1500 g (up to and including 1499 g).

Within the context of the present invention, the term "extremely low birth weight" means a birth weight of less than 1000 g (up to and including 999 g).

The term "small for gestational age infant" refers to infants having a birth weight that is more than 2 standard deviations below the mean reference to a birth weight for gestational growth chart or having a birth weight that is less than the 10^{th} percentile of population-based weight data obtained from infants at the same gestational age. The term "small for gestational age infants" includes infants who are small at birth either from a constitutive or genetic origin or, as a consequence of intrauterine growth restriction.

Within the context of the present invention, the term "young children" or "toddler" indicates a child between the age of 1 and 3 years.

The term "infant formula" as used herein refers to a nutritional composition intended for infants and as defined in *Codex Alimentarius,* (Codex STAN 72-1981) and Infant Specialities (incl. Food for Special Medical Purpose) as defined in *Codex Alimentarius,* (Codex STAN 72-1981). It also refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). The infant formulas encompass the starter infant formulas and the follow-up or follow-on formulas. Generally, a starter formula is for infants from birth as breast-milk substitute, and a follow-up or follow-on formula from the 6th month onwards.

The "growing-up milks" (or GUMs) are given from one year onwards. It is generally a milk-based beverage adapted for the specific nutritional needs of young children. They are nutritional compositions used for feeding children from 12 months to 2-3 years old in combination with other foods.

Within the context of the present invention, the term "fortifier"" refers to a composition which comprises one or more nutrients having a nutritional benefit for infants or young children.

By the term "milk fortifier", it is meant any composition used to fortify or supplement either human breast milk, infant formula, growing-up milk or human breast milk fortified with other nutrients. Accordingly, the human milk fortifier of the present invention can be administered after dissolution in human breast milk, infant formula, growing-up milk or human breast milk fortified with other nutrients or otherwise it can be administered as a standalone composition.

When administered as a stand-alone composition, the human milk fortifier of the present invention can be also identified as being a "supplement". In one embodiment, the milk fortifier of the present invention is a supplement.

By the term "human milk fortifier", it is meant any composition used to fortify or supplement human breast milk, or human breast milk fortified with other nutrients. The "human milk fortifier" according to the present invention may be intended to be administered to infants who were born preterm, with very low birth weight (VLBW) or with extremely low birth weight (ELBW).

The milk fortifier according to the present invention may be in powder or liquid form.

Milk fortifier compositions having a liquid form presents some particular benefits. For example, liquid formulations might be more convenient if coupled with a packaging that delivers calibrated drops of a certain weight or volume.

In addition, liquid formulations are easier to mix with the compositions to be fortified, whereas the powder ones can, in some cases, form lumps.

Within the context of the present invention, the term "nutritional composition" means a composition which nourishes a subject. This nutritional composition is usually to be taken orally or intravenously. It may include a lipid or fat source, a carbohydrate source and/or a protein source. In a particular embodiment the nutritional composition is a ready-to-drink composition such as a ready-to-drink formula.

Within the context of the present invention, the term 'increasing differentiation efficiency and maturation of mammalian induced pluripotent stem cell (miPSCs) into mammary-gland progenitor cells' means increasing the proportion of mammary-gland progenitor cells compared to non-mammary-gland progenitor cells, generated from a starting population of miPSCs which have undergone a differentiation protocol. In the present invention, said differentiation protocol includes the use of BMP4 and RA. Thus, said increase of the proportion of mammary-gland progenitor cells may be compared to the proportion of mammary-gland progenitor cells relative to non-mammary-gland progenitor cells, generated from a starting population of miPSCs which have undergone a differentiation protocol that does not include the use of BMP4 and RA but is otherwise the same.

Within the context of the present invention, the term 'increasing viability' means increasing the number of cells that are live and healthy. Said cells are capable of further differentiation steps, for example in the context of the present invention, are capable of developing into mammary organoids.

Within the context of the present invention, the term 'mammary-gland progenitor cells' or similar means cells that express at least two mammary-gland progenitor markers. Said markers include but are not limited to CD49f, EpCAM, MUC1 and GATA3. Conversely, within the context of the present invention, the term 'non mammary-gland progenitor cells' or similar means cells that do not express at least two mammary-gland progenitor markers.

Reference herein to EpiCult or EpiCultB medium refers to a serum free culture medium comprising hydrocortisone, insulin, FGF10 and HGF.

A culture medium as disclosed anywhere herein refers to a solid, semi-solid or liquid comprising essential nutrients, designed to support the growth and differentiation of microorganisms. MammoCult medium is one example of a culture medium that may be used in the present invention.

### Methods and Uses

The present invention relates to methods of producing mammary gland cells using iPSCs that are cultured and differentiated in specific conditions described below and using said mammary gland cells in methods for producing a human milk like product *in vitro,* wherein exosomes are purified from the human milk like product in order to isolate the exosomes.

It has been surprisingly demonstrated in the present invention that the addition of Bone Morphogenetic Protein 4 (BMP4) and/or Retinoic Acid (RA) in the methods as described anywhere herein increase the efficiency of the differentiation protocol and improve the yield and quality of the end milk like product. More specifically, it has been demonstrated that BMP4 and/or RA increase the differentiation efficiency of iPSCs to mammary gland progenitor cells, and thus increasing the number of mammary gland progenitor cells produced by the methods as described anywhere herein. The benefits of this include a higher yield of mammary gland cells. It has been demonstrated that BMP4 and RA improve expression and secretion levels of milk-specific bioactive markers such as osteopontin (OPN). It has also been demonstrated that because of the increased efficiency of differentiation due to the combination of BMP4 and RA, the overall differentiation protocol can be shortened in length. This has significant benefits including reduce cell death, higher milk like yields, and cost savings.

### Mammalian mammary cell production

In one aspect, the present invention relates to methods of producing mammary gland cells using iPSCs that are cultured and differentiated in specific conditions.

Thus, the invention provides a method of producing a population of mammary gland cells, comprising:
i) culturing mammalian induced pluripotent stem cells (miPSCs) in a culture medium comprising bone morphogenic protein 4 (BMP4) and/or retinoic acid (RA) to generate embryoid bodies (EBs), and
ii) growing the EBs to generate a population of mammary cells.

In some embodiments, the invention provides a method of producing a population of mammary gland cells, comprising:
i) culturing mammalian induced pluripotent stem cells (miPSCs) in a culture medium comprising bone morphogenic protein 4 (BMP4) to generate embryoid bodies (EBs), and
ii) growing the EBs to generate a population of mammary cells.

Thus, the invention provides a method of producing a population of mammary gland cells, comprising:
i) culturing mammalian induced pluripotent stem cells (miPSCs) in a culture medium comprising retinoic acid (RA) to generate embryoid bodies (EBs), and
ii) growing the EBs to generate a population of mammary cells.

In some embodiments, the invention provides a method of producing a population of mammary gland cells, comprising:
i) culturing mammalian induced pluripotent stem cells (miPSCs) in a culture medium comprising bone morphogenic protein 4 (BMP4) and retinoic acid (RA) to generate embryoid bodies (EBs), and
ii) growing the EBs to generate a population of mammary cells.

The invention also provides use of BMP4 and/or RA for increasing the differentiation efficiency of mammalian induced pluripotent stem cell (miPSCs) into mammary-gland progenitor cells in a differentiation protocol. In some embodiments, the invention also provides the use of BMP4 and/or RA for increasing the efficiency of producing a mammalian milk like product.

In some embodiments, the mammary gland cells are human mammary gland cells. In some embodiments, the mammary gland cells form lactocyte mammary-like gland organoids. Said lactocyte mammary-like gland organoids are lactogenic, i.e. are capable of producing milk.

In some embodiments, BMP4 is added to the culture medium in the early stages of the differentiation methods as described anywhere herein. In some embodiments, BMP4 is added to the culture medium between day 0 and day 10. In some embodiments, BMP4 is added to the culture medium between day 0 and day 6. In some embodiments, BMP4 is added to the culture medium between day 0 and day 3. Day 0 is the time point where the iPSCs are first added to the culture medium, i.e. when the iPSCs are first induced for differentiation.

In some embodiments, BMP4 is added to the culture medium for 3 days.

In some embodiments, BMP4 is added to the culture medium in a concentration of 5 to 20 ng/mL. In some embodiments, BMP4 is added to the culture medium in a concentration of 5 ng/mL. In some embodiments, BMP4 is added to the culture medium in a concentration of 10 ng/mL. In some embodiments, BMP4 is added to the culture medium in a concentration of 20 ng/mL.

In some embodiments, BMP4 is added to the culture medium between day 0 and day 3, and in a concentration of between 5 to 20 ng/mL.

In some embodiments, RA is added to the culture conditions in the early stages of the differentiation methods as described anywhere herein. In some embodiments, RA is added to the culture medium during the mammary lineage commitment stage. In some embodiments, RA is added to the culture medium between day 10 and day 15. In some embodiments, RA is added to the culture medium between day 6 and day 11. In some embodiments, RA is not added to the culture medium after day 11. In some embodiments, RA is added to the culture medium before day 11. Day 0 is the time point where the iPSCs are first added to the culture medium, i.e. when the iPSCs are first induced for differentiation.

In some embodiments, RA is added to the culture medium for 5 days.

In some embodiments, RA is added to the culture medium in a concentration of 1 uM.

In some embodiments, RA is added to the culture conditions between day 10 and day 15, and in a concentration of between 1 µM.

In some embodiments, BMP4 is added to the culture medium between day 0 and day 3, and RA is added to the culture medium between day 10 and day 15.

In some embodiments, BMP4 is added to the culture medium between day 0 and day 3, and RA is added to the culture medium between day 6 and day 11.

The EBs generated in the methods as described anywhere herein express one or more mammary gland positive progenitor-cell markers. In some embodiments, said one or more mammary gland positive progenitor-cell markers are selected from EpCAM, CD49f, MUC1 and GATA3.

In some embodiments, the EBs have increased expression of one or more mammary gland positive progenitor-cell markers compared to the expression level of said mammary gland positive progenitor-cell markers in EBs not treated with BMP4.

In some embodiments, at least 10%, 20%, 30%, 40%, 50%, 60%, 70%, 80% or more of the EBs express one or more mammary gland positive progenitor-cell markers. In some embodiments, at least 10%, 20%, 30%, 40%, 50%, 60%, 70%, 80% or more of the EBs express two or more mammary gland positive progenitor-cell markers. In some embodiments, said mammary gland positive progenitor-cell markers are selected from EpCAM, CD49f, MUC1 and GATA3.

In some embodiments, at least 35% of EBs express EpCAM and CD49f mammary gland positive progenitor-cell markers. In some embodiments, at least 60% of EBs express EpCAM and CD49f mammary gland positive progenitor-cell markers at the middle differentiation stage. In some embodiments, at least 35% of EBs express EpCAM and CD49f mammary gland positive progenitor-cell markers at the pre-induction stage. In some embodiments, at least 15% of EBs express EpCAM and CD49f mammary gland positive progenitor-cell markers at the post-induction stage. In some embodiments, at least 40% of EBs express EpCAM and CD49f mammary gland positive progenitor-cell markers at the post-induction stage.

In some embodiments, at least 15% of EBs express MUC1 and EpCAM mammary gland positive progenitor-cell markers. In some embodiments, at least 20% of EBs express MUC1 and EpCAM mammary gland positive progenitor-cell markers at the middle differentiation stage. In some embodiments, at least 15% of EBs express MUC1 and EpCAM mammary gland positive progenitor-cell markers at the pre-induction stage. In some embodiments, at least 5% of EBs express MUC1 and EpCAM mammary gland positive progenitor-cell markers at the post-induction stage. In some embodiments, at least 15% of EBs express MUC1 and EpCAM mammary gland positive progenitor-cell markers at the post-induction stage.

In some embodiments, at least 20% of EBs express GATA3 and EpCAM mammary gland positive progenitor-cell markers. In some embodiments, at least 50% of EBs express GATA3 and EpCAM mammary gland positive progenitor-cell markers at the middle differentiation stage. In some embodiments, at least 25% of EBs express GATA3 and EpCAM mammary gland positive progenitor-cell markers at the pre-induction stage. In some embodiments, at least 15% of EBs express GATA3 and EpCAM mammary gland positive progenitor-cell markers at the post-induction stage. In some embodiments, at least 40% of EBs express GATA3 and EpCAM mammary gland positive progenitor-cell markers at the post-induction stage.

In some embodiments, the middle differentiation stage is day 25, the pre-induction stage is day 35 and the post-induction stage is day 42. In some embodiments, the middle differentiation stage is between day 20. The pre-induction stage is day 26 and the post-induction stage is day 31.

In some embodiments, the EBs express one or more milk-specific bioactive markers. In some embodiments, said milk-specific bioactive marker is osteopontin (OPN).

In some embodiments, the EBs have increased expression of one or more milk-specific bioactive markers compared to the expression level of said markers in EBs not treated with BMP4 and/or RA. In some embodiments, the EBs have increased secretion of one or more milk-specific bioactive markers compared to the expression level of said markers in EBs not treated with BMP4 and/or RA. In some embodiments, the EBs have increased secretion of OPN compared to the expression level of OPN in EBs not treated with BMP4 and/or RA. In some embodiments, the EBs have 40% or more increased secretion of OPN compared to the expression level of OPN in EBs not treated with BMP4 and/or RA.

The methods of producing a population of mammary gland cells as described anywhere herein, may be combined and/or utilized in the methods for producing a mammalian milk like product as described anywhere herein, particular as part of Step A) as described anywhere herein.

For example, in some embodiments of the method of producing a population of mammary gland cells as described anywhere herein, the culturing step i) comprises culturing the miPSCs in MammoCult medium and BMP4, in a 3D-suspension culture system, for example 3D-suspension condition, thereby directing the iPSCs to differentiate towards non-neural ectoderm cells. In some embodiments, step A i) is for at least 12 days. In some embodiments, step A i) is for no more than 8 days.

In some embodiments in some embodiments of the method of producing a population of mammary gland cells as described anywhere herein, the growing step ii) comprises growing the EBs in a 3D embedding system comprising RA, for example a mixed floating gel composed of matrix protein such as Matrigel and/or Collagen I. In some embodiments, step A ii) is for at least 30 days, for example for 32 days. In some embodiments, step A ii) is for at least 23 days, for example no more than 25 days.

### Mammalian milk like product production

In another aspect, the present invention relates to methods for producing a mammalian milk like product as defined herein, including any of steps A) and B) as defined herein and optional step C) as defined herein. Said methods for producing a mammalian milk like product as defined herein may also include the methods of producing mammalian mammary cells, as part of step A). In particular, BMP4 and/or RA may be added to any of the methods for producing a mammalian milk like product as defined herein, particularly as part of step A). Said methods also include different lengths of time for producing the mammalian milk like product.

### Step A - Generating lactocytes and/or mammary like organoids from hiPSCs

According to the method of the present invention, mammary like cells and/or organoid structures are generated under step A).

Such mammary like cells and/or organoid structures can be generated according to any reported method making use of iPSC.

In one embodiment, such mammary like cells and/or organoid structures may be generated according to the procedure described in Ying Qu et al., Stem Cell Reports, Vol.8, 205-215 which is hereby incorporated in its entirety.

More precisely, the methodology described in the above-mentioned scientific publication (herein-below also referred to as "Ying Qu publication", or Ying Qu, et al. (2017)) represents a two-step protocol to generate human mammary like cells and/or organoids from iPSCs.

It preferably includes as a first step (step 1) the differentiation and enrichment of non-neural ectoderm-cell-containing spheres (mEBs/mammospheres) from iPSCs and as a second step (step 2) the generation of mammary like organoids from 10-day mEBs (mammospheres) using 3D floating mixed gel-culture of Matrigel and Collagen I.

More specifically, step A preferably includes:
- Embryoid bodies formation
- Mammary lineage commitment
- Branch and alveolar differentiation, and
- Induction of milk bioactives.

Each of these stages may be conducted for a specific amount of time, with specific culture mediums.

In some embodiments, step A is conducted for a total of 40 to 45 days, preferably 42 days. In some embodiments, step A is conducted for a total of 40 to 42 days. In some embodiments, step A is conducted for 41 days

In some embodiments, step A is shortened and is conducted for less than 42 days, optionally less than 40 days, optionally less than 31 days. Preferably step A is conducted for 31 days. In some embodiments, step A is conducted for 30 to 39 days. In some embodiments, step A is conducted for 35 to 39 days. In some embodiments, step A is conducted for 30 to 35 days.

In some embodiments, the embryoid bodies formation stage is between day 0 and day 10. In some embodiments, the embryoid bodies formation stage is for 10 days.

In some embodiments, the embryoid bodies formation stage is shortened and is between day 0 and day 6. In some embodiments, the embryoid bodies formation stage is for 6 days. In some embodiments, the embryoid bodies formation stage is for 10 days or less.

In some embodiments, the mammary lineage commitment stage is between day 10 and day 15. In some embodiments, the mammary lineage commitment stage is between day 6 and day 11. In some embodiments, the embryoid bodies formation stage is for 5 days. In some embodiments, the embryoid bodies formation stage is for no more than 5 days.

In some embodiments, the branch and alveolar differentiation stage is between day 15 and day 35. In some embodiments, the branch and alveolar differentiation stage is for 20 days.

In some embodiments, the branch and alveolar differentiation stage is shortened and is between day 11 and day 26. In some embodiments, the branch and alveolar differentiation stage is for 15 days. In some embodiments, the branch and alveolar differentiation stage is for no more than 15 days.

In some embodiments, the induction of milk bioactive stage is between day 35 and day 42. In some embodiments, the induction of milk bioactive stage is for 7 days.

In some embodiments, the induction of milk bioactive stage is shortened and is between day 26 and day 31. In some embodiments, the induction of milk bioactive stage is for 5 days. In some embodiments, the induction of milk bioactive stage is for no more than 5 days.

Thus, in some embodiments, step A is conducted for a total of 42 days, wherein the embryoid bodies formation stage is between day 0 and day 10, the mammary lineage commitment stage is between day 10 and day 15, the branch and alveolar differentiation stage is between day 15 and day 35, and the induction of milk bioactive stage is between day 35 and day 42.

In other embodiments, the process is shortened such that step A is conducted for a total of 31 days, wherein the embryoid bodies formation stage is between day 0 and day 6, the mammary lineage commitment stage is between day 6 and day 11, the branch and alveolar differentiation stage is between day 11 and day 26, and the induction of milk bioactive stage is day 26 and day 31.

Further details of the time periods and culture mediums for use in the methods described anywhere herein are provided below.

In step 1 differentiation and enrichment of non-neural ectoderm-cell-containing spheres (mEBs/mammospheres) from hiPSCs occurs by culturing hiPSCs in complete MammoCult medium (StemCell Technologies). Complete MammoCult medium is preferably composed of the basal medium, proliferation supplements, heparin (typically 4µg/mL), and hydrocortisone (typically 0.48µg/mL). Medium is usually changed every three days. mEBs (mammospheres) obtained in said step are then enriched for non-neural ectoderm cells.

In some embodiments, BMP4 is added to the culture medium in step 1.

In some embodiments, BMP4 is added to the culture medium as described in anywhere herein.

In some embodiments, BMP4 is added to the culture conditions in the early stages of the differentiation methods as described anywhere herein. In some embodiments, BMP4 is added to the culture medium between day 0 and day 10. In some embodiments, BMP4 is added to the culture medium between day 0 and day 6. In some embodiments, BMP4 is added to the culture medium between day 0 and day 3. Day 0 is the time point where the iPSCs are first added to the culture medium, i.e. when the iPSCs are first induced for differentiation.

In some embodiments, BMP4 is added to the culture medium for 3 days.

In some embodiments, BMP4 is added to the culture medium in a concentration of 5 to 20 ng/mL. In some embodiments, BMP4 is added to the culture medium in a concentration of 5 ng/mL. In some embodiments, BMP4 is added to the culture medium in a concentration of 10 ng/mL. In some embodiments, BMP4 is added to the culture medium in a concentration of 20 ng/mL.

In some embodiments, BMP4 is added to the culture medium between day 0 and day 3, and in a concentration of between 5 to 20 ng/mL.

In step 2, following the protocol of Ying Qu, et al. (2017), mammary-like organoids are generated by firstly preparing a 3D culture on basis of a floating mixed gel (e.g. Matrigel and Collagen I). 10 day mEBs (mammospheres) are then grown for 5 days in the mixed gel floated in complete EpiCultB medium supplemented with Parathyroid hormone (pTHrP). For induction of branch and alveolar differentiation for preparation of mammary like organoids/lactocytes, cells are then cultured in complete EpiCultB medium supplemented with hydrocortisone, insulin, FGF10 and HGF. Milk protein expression is typically induced at day 35 by adding prolactin, hydrocortisone and insulin to complete EpiCultB medium supplemented with BSA (lactogenic medium) and culturing for 5 days. The process of Ying Qu, et al. (2017) is typically completed at day 40.

In some embodiments, RA is added to the culture medium in step 2.

In some embodiments, RA is added to the culture medium as described in anywhere herein.

In some embodiments, RA is added to the culture medium in the early stages of the differentiation methods as described anywhere herein. In some embodiments, RA is added to the culture medium during the mammary lineage commitment stage. In some embodiments, RA is added to the culture medium between day 10 and day 15. In some embodiments, RA is added to the culture medium between day 6 and day 11. In some embodiments, RA is not added to the culture medium after day 11. Day 0 is the time point where the iPSCs are first added to the culture medium, i.e. when the iPSCs are first induced for differentiation.

In some embodiments, RA is added to the culture medium for 5 days.

In some embodiments, RA is added to the culture medium in a concentration of 1 µM.

In some embodiments, RA is added to the culture medium between day 10 and day 15, and in a concentration of between 1 µM.

In one embodiment of the present invention, a method for producing a human milk like product is provided comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, and after 10 days collecting mammospheres formed thereof; and
ii) growing such mammospheres in an appropriate system comprising RA as described anywhere herein (for example a floating mixed gel culture system as described in Hassiotou F. et al. Stem Cells. 2012) for at least 10 days to generate lactocytes.

In one embodiment of the present invention, a method for producing a human milk like product is provided comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, and after 6 days collecting mammospheres formed thereof; and
ii) growing such mammospheres in an appropriate system comprising RA as described anywhere herein (for example a floating mixed gel culture system as described in Hassiotou F. et al. Stem Cells. 2012) for less than 10 days to generate lactocytes.

In another embodiment, a method for producing a human milk like product is provided comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, in non-adherent conditions for mammospheres formation; and
ii) growing such mammospheres in a 3D appropriate system comprising RA as described anywhere herein (for example a mixed floating gel composed of matrix protein such as Matrigel and/or Collagen or in suspension cultures in non-adherent plates) for at least 10 days to generate lactocytes.

In another embodiment, a method for producing a human milk like product is provided comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, in non-adherent conditions for mammospheres formation; and
ii) growing such mammospheres in a 3D appropriate system comprising RA as described anywhere herein (for example a mixed floating gel composed of matrix protein such as Matrigel and/or Collagen or in suspension cultures in non-adherent plates) for less than 10 days to generate lactocytes.

In one embodiment, mammary commitment under step A) is obtained by applying a conditioned medium (for example EpiCultB) supplemented with specific factors (for example Parathyroid hormone (pTHrP), hydrocortisone, insulin, FGF10, and HGF) and RA.

In one embodiment of the present invention, the method comprises generating mammary - like organoids under step A).

In one embodiment of the present invention, the method to generate mammary - like organoids under step A) includes culturing the cells under conditions selected from the group consisting of: 2D monolayers of cells, 2D with attached EBs, in suspension in non-adherent plates and in mixed floating gel.

In a preferred embodiment, mixed floating gel comprises Matrigel and Collagen I.

In another preferred embodiment, mammospheres (mEBs) in step A) are grown in an appropriate system (for example a floating mixed gel culture system as described in Hassiotou F. et al. Stem Cells. 2012) for at least 15 days.

In a more preferred embodiment, mammospheres (mEBs) in step A) are grown in an appropriate system (for example a floating mixed gel culture system as described in Hassiotou F. et al. Stem Cells. 2012) for 20 days.

In one embodiment, the method according to the present invention provides for culture conditions according to step A) [for example under step A)i) and /or under step A)ii)], which are adapted to generate lactocytes derived from human induced pluripotent stem cells (hiPSC) capable to secrete a human milk like product.

In a preferred embodiment, a method for producing a human milk like product is provided comprising generating lactocytes under step A) from human induced pluripotent stem cells (hiPSC), where such step A) comprises directing hiPSCs to differentiate towards mammary gland cells (for example lactocytes) in an appropriate 3D culture system comprising BMP4 and/or RA as described anywhere herein (for example 3D-suspension condition). In some embodiments, for at least 42 days. In some embodiments, for no more than 31 days. In some embodiments, for at least 31 days.

In another preferred embodiment, a method for producing a human milk like product is provided comprising generating lactocytes under step A) from human induced pluripotent stem cells (hiPSC), where such step A) comprises:
i) directing hiPSCs to differentiate towards non-neural ectoderm cells by culturing them in an appropriate culture medium (for example MammoCult medium) and BMP4 as described anywhere herein, in an appropriate 3D culture system (for example 3D-suspension condition) for at least 12 days (day -2 to day 10), and
ii) growing the formed mEBs (mammospheres) in an appropriate 3D embedding system comprising RA as described anywhere herein (for example a mixed floating gel composed of matrix protein such as Matrigel and/or Collagen I) for at least 30 days, preferably for 32 days, to generate lactocytes.

In another preferred embodiment, a method for producing a human milk like product is provided comprising generating lactocytes under step A) from human induced pluripotent stem cells (hiPSC), where such step A) comprises:
i) directing hiPSCs to differentiate towards non-neural ectoderm cells by culturing them in an appropriate culture medium (for example MammoCult medium) and BMP4 as described anywhere herein, in an appropriate 3D culture system (for example 3D-suspension condition) for no more than 8 days (day -2 to day 6), and
ii) growing the formed mEBs (mammospheres) in an appropriate 3D embedding system comprising RA as described anywhere herein (for example a mixed floating gel composed of matrix protein such as Matrigel and/or Collagen I) for no more than 25 days, to generate lactocytes.

In a particularly preferred embodiment of the present invention, a method of producing a human milk like product is provided comprising generating lactocytes under step A) from human induced pluripotent stem cells (hiPSCs), wherein step A)i) is defined as follows:
i) generation of embryoid bodies (EBs) from hiPSCs by incubation in standard iPSC medium E8 (comprising DMEM/F12, L-ascorbic acid-2-phosphate magnesium, sodium selenium, FGF2, insulin, NaHCO₃ and transferrin, TGFβ1 or NODAL as described in Chen et al., Nat Methods, 2011) or mTeSR^{™} for two days (day -2-day 0), and producing mEBs (mammospheres) highly enriched in non-neural ectodermal cells by incubation of EBs in complete MammoCult medium (StemCell Technologies) comprising the basal medium, proliferation supplement and supplemented with heparin (typically 4µg/mL), hydrocortisone (typically 0.48µg/mL) and BMP4 as described anywhere herein, for 10 days (day 0-day 10), and wherein
   step A)ii) is distinguished into further substeps and comprises the following steps: ii), iii) and iv):
ii) incubation of mEBs (mammospheres) in complete EpiCultB medium supplemented with EpiCult proliferation supplement and Parathyroid hormone (pTHrP) for 5 days (day 10-day 15),
iii) promotion of branch and alveolar differentiation and mammary cell specification by incubating mEBs (mammospheres) in EpiCultB medium supplemented with EpiCult proliferation supplement, hydrocortisone, insulin, FGF10 and HGF for 20 days (day 15-day 35), and
iv) induction of milk protein expression by incubating mEBs (mammospheres) in EpiCultB medium supplemented with EpiCult proliferation supplement, hydrocortisone, insulin, FBS, prolactin, progesterone and β-estradiol for 7 days (day 35-day 42).

In a particularly preferred embodiment of the present invention, a method of producing a human milk like product is provided comprising generating lactocytes under step A) from human induced pluripotent stem cells (hiPSCs), wherein step A)i) is defined as follows:
i) generation of embryoid bodies (EBs) from hiPSCs by incubation in standard iPSC medium E8 (comprising DMEM/F12, L-ascorbic acid-2-phosphate magnesium, sodium selenium, FGF2, insulin, NaHCO₃ and transferrin, TGFβ1 or NODAL as described in Chen et al., Nat Methods, 2011) or mTeSR^{™} for two days (day -2-day 0), and producing mEBs (mammospheres) highly enriched in non-neural ectodermal cells by incubation of EBs in complete MammoCult medium (StemCell Technologies) comprising the basal medium, proliferation supplement and supplemented with heparin (typically 4µg/mL), hydrocortisone (typically 0.48µg/mL) and BMP4 as described anywhere herein, for 6 days (day 0-day 6), and wherein
   step A)ii) is distinguished into further substeps and comprises the following steps: ii), iii) and iv):
ii) incubation of mEBs (mammospheres) in complete EpiCultB medium supplemented with EpiCult proliferation supplement and Parathyroid hormone (pTHrP) and RA for 5 days (day 6-day 11),
iii) promotion of branch and alveolar differentiation and mammary cell specification by incubating mEBs (mammospheres) in EpiCultB medium supplemented with EpiCult proliferation supplement, hydrocortisone, insulin, FGF10 and HGF for 15 days (day 11-day 26), and
iv) induction of milk protein expression by incubating mEBs (mammospheres) in EpiCultB medium supplemented with EpiCult proliferation supplement, hydrocortisone, insulin, FBS, prolactin, progesterone and β-estradiol for 5 days (day 26-day 31).

Step iv) preferably leads to differentiation into milk protein expressing cells, particularly lactocytes, and/or mammary like gland organoids.

In a further particularly preferred embodiment of the present invention, a method of producing a human milk like product is provided comprising generating lactocytes under step A) from human induced pluripotent stem cells (hiPSCs), wherein step A)i) is defined as follows:
i) generation of embryoid bodies (EBs) from hiPSCs by incubation in standard iPSC medium E8 (comprising DMEM/F12, L-ascorbic acid-2-phosphate magnesium, sodium selenium, FGF2, insulin, NaHCO₃ and transferrin, TGFβ1 or NODAL as described in Chen et al., Nat Methods, 2011) mTeSR^{™} for two days (day-2-day 0), and producing mEBs (mammospheres) highly enriched in non-neural ectodermal cells by incubation of EBs in MammoCultB medium supplemented with MammoCult proliferation supplement, hydrocortisone, heparin and BMP4 as described anywhere herein, for 10 days (day 0-day 10), and wherein step A)ii) is distinguished into further substeps and comprises the following steps ii), iii) and iv):
ii) embedding the formed mEBs (mammospheres) in a mixture of Matrigel and Collagen I floated in EpiCultB medium supplemented with EpiCult proliferation supplement and Parathyroid hormone (pTHrP) and RA for 5 days (day 10-day 15),
iii) promotion of branch and alveolar differentiation and mammary cell specification by incubating embedded mEBs (mammospheres) in EpiCultB medium supplemented with EpiCult proliferation supplement, hydrocortisone, insulin, FGF10 and HGF for 20 days (day 15 to day 35), and
iv) induction of milk protein expression by incubating mEBs (mammospheres) in EpiCultB medium supplemented with EpiCult proliferation supplement, hydrocortisone, insulin, FBS, prolactin, progesterone and β-estradiol for 7 days (day 35 to day 42).

In a further particularly preferred embodiment of the present invention, a method of producing a human milk like product is provided comprising generating lactocytes under step A) from human induced pluripotent stem cells (hiPSCs), wherein step A)i) is defined as follows:
i) generation of embryoid bodies (EBs) from hiPSCs by incubation in standard iPSC medium E8 (comprising DMEM/F12, L-ascorbic acid-2-phosphate magnesium, sodium selenium, FGF2, insulin, NaHCO₃ and transferrin, TGFβ1 or NODAL as described in Chen et al., Nat Methods, 2011) mTeSR^{™} for two days (day-2-day 0), and producing mEBs (mammospheres) highly enriched in non-neural ectodermal cells by incubation of EBs in MammoCultB medium supplemented with MammoCult proliferation supplement, hydrocortisone, heparin and BMP4 as described anywhere herein, for 6 days (day 0-day 6), and wherein step A)ii) is distinguished into further substeps and comprises the following steps ii), iii) and iv):
ii) embedding the formed mEBs (mammospheres) in a mixture of Matrigel and Collagen I floated in EpiCultB medium supplemented with EpiCult proliferation supplement and Parathyroid hormone (pTHrP) and RA for 5 days (day 6-day 11),
iii) promotion of branch and alveolar differentiation and mammary cell specification by incubating embedded mEBs (mammospheres) in EpiCultB medium supplemented with EpiCult proliferation supplement, hydrocortisone, insulin, FGF10 and HGF for 15 days (day 11 to day 26), and
iv) induction of milk protein expression by incubating mEBs (mammospheres) in EpiCultB medium supplemented with EpiCult proliferation supplement, hydrocortisone, insulin, FBS, prolactin, progesterone and β-estradiol for 5 days (day 26 to day 31).

Step iv) preferably leads to differentiation into milk protein expressing cells, particularly lactocytes, and/or mammary like gland organoids.

Standard iPSC medium E8 (comprising DMEM/F12, L-ascorbic acid-2-phosphate magnesium, sodium selenium, FGF2, insulin, NaHCO3 and transferrin, TGFβ1 or NODAL as described in Chen et al., Nat Methods, 2011) as mentioned herein is commercially available, e.g., as "Essential 8^{™} Medium" from ThermoFischer Scientific, catalogue number A1517001 (see also https:/ /www.thermofisher.com/order/catalog/product/ A1517001#/ A1517001).

mTeSR^{™} medium is commercially available from STEMCELL Technologies, catalogue number 85850 (see also https://www.stemcell.com/mtesrl.html). Suchc medium is also described in "Defined, Feeder- Independent medium for human hembryonic stem cell culture", Current protocol in Stem Cell Biology, Volume 2, Issue 1, Sept 2007.

In one embodiment, steps iii) and/or iv) as defined above for the particularly preferred embodiments, preferably lead to formation of/differentiation into at least breast cells, luminal cells, and basal cells. In this context, breast cells preferably express one or more, preferably all of markers selected from the group consisting of: β-Casein, milk protein, and hormone receptors. Moreover, luminal cells preferably express one or more, preferably all markers selected from the group consisting of: EpCAM, MUC1, CD49F, GATA3, CK8, and CK18. Moreover, basal cells preferably express one or more markers selected from the group consisting of: CK14, α-smooth muscle actin and P63.

In one further embodiment, after induction of mEBs (mammospheres) in step ii) and/or iv) as defined above for the particularly preferred embodiments, mammary like gland organoids may be obtained, that express one or more markers selected from the group consisting of: β-Casein, milk protein, and hormone receptors, luminal cells that express one or more markers selected from the group consisting of: EpCAM, MUC1, CD49F, GATA3, CK8, CK18, and basal cells that express one or more markers selected from the group consisting of: CK14, α-smooth muscle actin and P63.

In one embodiment of the invention, the methods above described are provided for producing a human milk like product.

In one embodiment (of step A), delivery of nutrients and biomimetic stimuli is controlled to influence cell growth, differentiation and tissue formation. In one embodiment (of step A), such control is performed in a bioreactor.

Where BMP4 and/or RA are added to the culture medium in the methods for producing a mammalian milk like product as described anywhere herein, the EBs generated in the methods as described anywhere herein express one or more mammary gland positive progenitor-cell markers. In some embodiments, said one or more mammary gland positive progenitor-cell markers are selected from EpCAM, CD49f, MUC1 and GATA3.

In some embodiments, the EBs have increased expression of one or more mammary gland positive progenitor-cell markers compared to the expression level of said mammary gland positive progenitor-cell markers in EBs not treated with BMP4.

In some embodiments, at least 10%, 20%, 30%, 40%, 50%, 60%, 70%, 80% or more of the EBs express one or more mammary gland positive progenitor-cell markers. In some embodiments, at least 10%, 20%, 30%, 40%, 50%, 60%, 70%, 80% or more of the EBs express two or more mammary gland positive progenitor-cell markers. In some embodiments, said mammary gland positive progenitor-cell markers are selected from EpCAM, CD49f, MUC1 and GATA3.

In some embodiments, at least 35% of EBs express EpCAM and CD49f mammary gland positive progenitor-cell markers. In some embodiments, at least 60% of EBs express EpCAM and CD49f mammary gland positive progenitor-cell markers at the middle differentiation stage. In some embodiments, at least 35% of EBs express EpCAM and CD49f mammary gland positive progenitor-cell markers at the pre-induction stage. In some embodiments, at least 40% of EBs express EpCAM and CD49f mammary gland positive progenitor-cell markers at the post-induction stage.

In some embodiments, at least 15% of EBs express MUC1 and EpCAM mammary gland positive progenitor-cell markers. In some embodiments, at least 20% of EBs express MUC1 and EpCAM mammary gland positive progenitor-cell markers at the middle differentiation stage. In some embodiments, at least 15% of EBs express MUC1 and EpCAM mammary gland positive progenitor-cell markers at the pre-induction stage. In some embodiments, at least 5% of EBs express MUC1 and EpCAM mammary gland positive progenitor-cell markers at the post-induction stage.

In some embodiments, at least 20% of EBs express GATA3 and EpCAM mammary gland positive progenitor-cell markers. In some embodiments, at least 50% of EBs express GATA3 and EpCAM mammary gland positive progenitor-cell markers at the middle differentiation stage. In some embodiments, at least 25% of EBs express GATA3 and EpCAM mammary gland positive progenitor-cell markers at the pre-induction stage. In some embodiments, at least 15% of EBs express GATA3 and EpCAM mammary gland positive progenitor-cell markers at the post-induction stage.

In some embodiments, the middle differentiation stage is day 25. The pre-induction stage is day 35 and the post-induction stage is day 42. In some embodiments, the middle differentiation stage is between day 20. The pre-induction stage is day 26 and the post-induction stage is day 31.

In some embodiments, the EBs express one or more milk-specific bioactive markers. In some embodiments, said milk-specific bioactive marker is osteopontin (OPN).

In some embodiments, the EBs have increased expression of one or more milk-specific bioactive markers compared to the expression level of said non-neuronal ectodermal markers in EBs not treated with BMP4 and/or RA.

Thus, as an example, provided herein is a method for producing a human milk like product, comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, and after 10 days collecting mammospheres formed thereof; and
ii) growing such mammospheres in an appropriate system (for example a floating mixed gel culture system as described in Hassiotou F. et al. Stem Cells. 2012) for at least 10 days to generate lactocytes,
wherein the mammospheres (EBs) have increased expression of one or more mammary gland positive progenitor-cell markers compared to the expression level of said mammary gland positive progenitor-cell markers in mammospheres (EBs) not treated with BMP4.

Thus, as an example, provided herein is a method for producing a human milk like product, comprising generating lactocytes under step A) from human induced pluripotent stem cells (hiPSC), where such step A) comprises:
i) directing iPSCs to differentiate towards non-neural ectoderm cells by culturing them in an appropriate culture medium (for example MammoCult medium) and BMP4 as described anywhere herein, and after 6 days collecting mammospheres formed thereof; and
ii) growing such mammospheres in an appropriate system comprising RA (for example a floating mixed gel culture system as described in Hassiotou F. et al. Stem Cells. 2012) for less than 10 days to generate lactocytes,
wherein the mammospheres (EBs) have increased expression of one or more mammary gland positive progenitor-cell markers compared to the expression level of said mammary gland positive progenitor-cell markers in mammospheres (EBs) not treated with BMP4 and/or RA.

It will be understood that any method or method step disclosed herein may be conducted in 3D suspension culture rather than using a membrane matrix as a support. Thus, in some embodiments, during all the differentiation procedure, cells are maintained in suspension culture.

### Step B - Expressing a human breast milk like product

In one embodiment of the present invention, the methods comprise expressing the human milk like product from mammary like organoids derived from human induced pluripotent stem cells (hiPSCs), preferably prepared according to step A). Expressing human milk like products preferably occurs upon induction of expression of the human milk like product from such lactocytes and/or mammary-like gland organoids.

In one embodiment, lactating lactocytes are induced by applying a specific medium (for example EpiCultB) supplemented with lactogenic factors (for example prolactin, hydrocortisone, and insulin).

Particularly, the human milk like product obtained from mammary like organoids derived from human induced pluripotent stem cells (hiPSCs), preferably prepared according to step A), contains bioactives of human milk, selected from the group comprising or consisting of proteins, lipids or oligosaccharides, preferably human milk oligosaccharides, etc. Inventors particularly managed to identify with the particularly preferred protocol according to steps A i) to iv) as carried out above inter alia oligosaccharides (including lactose and some HMOs), lipids (including 4 fatty acids), proteins (7 detected including caseins), and miRNA (75 detected, including 11 typically detected in HBM).

In one embodiment, the human milk like product obtained from mammary like organoids derived from human induced pluripotent stem cells (hiPSCs), preferably prepared according to step A), contains bioactives of human milk, selected from the group comprising or consisting of: oligosaccharides, lipids, proteins, exosomes and miRNA. In one embodiment, the human milk like product obtained from mammary like organoids derived from human induced pluripotent stem cells (hiPSCs), preferably prepared according to step A), contains exosomes.

In another embodiment, human milk like product obtained from mammary like organoids derived from human induced pluripotent stem cells (hiPSCs), preferably prepared according to step A), contains bioactives of human milk, selected from the group comprising or consisting of: lactose, 6'SL, C-4:0 fatty acid, C-8:0 fatty acid, C-10:0 fatty acid, C-14:0 fatty acid, C-15:0 fatty acid, C-16:0 fatty acid, C-16:1n7 fatty acid, C-17:0 fatty acid, C-18:0 fatty acid, C-18:1 n9 fatty acid, C-18:1 fatty acid, C-18:2 n6 fatty acid, C-20:0 fatty acid, C-20:1 n9 fatty acid, C-18:3 n3 fatty acid, C-22:0 fatty acid, lactoferrin, albumin, prolactin, Alpha S1-casein, Hemoglobin subunit beta, Hemoglobin subunit alpha, α-lactalbumin, Alpha-2-macroglobulin, β-casein, bile salt-activated lipase, κ-casein, lactadherin, CD14, fatty acid synthase, IgA, plgR, Serum albumin, Xanthine dehydrogenase, exosomes, miR-21-5p, miR-181a-5p, miR-30d-5p, miR-30b-5p, miR-22-3p, miR-146b-3p, miR-30c-5p, miR-30a-5p, miR-30e-5p and miR-148b-3p.

In one embodiment of the present invention, the human milk like product obtained from mammary like organoids derived human induced pluripotent stem cells (hiPSCs) is a standard human milk product. In another embodiment of the present invention, the human milk like product obtained from mammary like organoids derived from human induced pluripotent stem cells (hiPSCs), is a non -standard human milk product.

### Step C - Further treatments to produce modified human breast milk like product

In one optional embodiment of the present invention, the herein-described methods comprise an additional step C) which is performed on the human milk like product obtainable from step B) and which comprises performing an additional treatment on such product to provide a modified human milk like product.

In one particular embodiment, the additional treatment step C) performed on the inventive human breast milk like product may be selected from the group consisting of: a purification step, an isolation process, an extraction process, a fractionation step, an enrichment process, an enzymatic treatment, the addition of further components (for example which can't be expressed by the human mammary gland organoid (such as for example Immunoglobulins, probiotic and/or minerals) or combinations thereof.

In particular, in some embodiments, the human milk like product described anywhere herein is extracted, isolated and purified as part of the methods described anywhere herein. Thus, the invention provides an isolated human milk like product.

In some embodiments, the isolated human milk like product is formulated into a composition. In some embodiments, the isolated human milk like product is formulated into a nutritional composition.

In some embodiments, particular bioactives of the human milk like product, selected from the group comprising or consisting of: oligosaccharides, lipids, proteins, exosomes and miRNA, are extracted, isolated and purified from the human milk like product. In particular, exosomes are extracted, isolated and purified from the human milk like product. Thus, the invention provides isolated bioactives of the human milk like product, selected from the group comprising or consisting of: oligosaccharides, lipids, proteins, exosomes and miRNA. In particular, the invention provides isolated exosomes.

In some embodiments, the isolated bioactives of the human milk like product are formulated into a composition. In some embodiments, the isolated bioactives of the human milk like product are formulated into a nutritional composition.

In some embodiments, the isolated exosomes of the human milk like product are formulated into a composition. In some embodiments, the isolated exosomes of the human milk like product are formulated into a nutritional composition.

In some embodiments, step C) comprises purifying exosomes from the human milk like product. In some embodiments, step C) comprises purifying exosomes from the human milk like product to remove impurities, for example by chromatography or filtration or ultracentrifugation, in order to isolate the exosomes. In some embodiments, step C) comprises purifying exosomes from the human milk like product by chromatography in orderto isolate the exosomes. In some embodiments, step C) comprises purifying exosomes from the human milk like product by filtration in order to isolate the exosomes. In some embodiments, step C) comprises purifying exosomes from the human milk like product by ultracentrifugation in order to isolate the exosomes.

In some embodiments, the chromatography is column-based chromatography, for example size inclusion or size exclusion chromatography.

It will be understood that a product obtained from a purified compared to a non-purified protocol will be different in terms of its composition. In particular, the surface composition of the product will be different as components such as proteins that are attached to the surface of a product molecule may be removed during the purification steps. For example, proteins present on the surface of the exosomes may be removed.

In some embodiments, step C) further comprises sterilising the isolated exosomes.

In some embodiments, step C) further comprises storing the isolated exosomes at below freezing, optionally at -80°C.

In some embodiments, step C) comprises formulating the isolated exosomes into a powder form, for example by freeze drying or spray drying, or a liquid form.

In some embodiments, the method further comprises formulating the isolated exosomes with supplementary nutritional ingredients.

In some embodiments, the method further comprises dispensing the isolated exosomes into a container for consumption.

### Human milk like products

### 'Standard' human breast milk like product

In one embodiment of the present invention, the human breast milk like product is a 'standard' human breast milk like product, i.e., comprises the same components as human breast milk of a well-nourished mother.

The benefits of breast feeding are well known in the scientific literature and the possibility to have access to human breast milk like product allows its use for a number of equally well-known health benefits.

In such an embodiment, the human breast milk like product can be used as a substitute of breastfeeding under circumstances where real breastfeeding is not possible.

In such embodiment, the human breast milk like product is intended to be used for example to support longer breastfeeding experience for women who have less milk or who stop to produce milk after 6 months from birth.

Similarly, the human breast milk like product is intended to be used for example to allow breastfeeding even under circumstances where sicknesses compromise real breastfeeding from the mother.

In another embodiment, the human breast milk like product is intended to be used under circumstances whereby breastmilk production would not naturally be initiated, for example if an infant is adopted.

In one embodiment, the human milk like product according to the present invention is not the product of human breast milk lactation as occurring in nature.

In one embodiment, the human breast milk like product is for use in providing optimal nutrition for infant.

In one embodiment, the human breast milk like product is for use in providing healthy growth in infants.

In one embodiment, the human breast milk like product is for use in preventing infection, obesity and promoting immunity development in infants.

In one embodiment, the human breast milk like product is a non-modified human breast milk like product.

In another embodiment, the human breast milk like product is a modified human breast milk like product.

In one embodiment, the human milk like product according to the present invention comprises: proteins, lipids, carbohydrates, vitamins and minerals.

In another embodiment, the human milk like product according to the present invention comprises: proteins, lipids, carbohydrates, vitamins, minerals and bioactives.

In one embodiment, the human milk like product according to the present invention comprises: proteins, lipids (including linoleic acid and alpha-linolenic acid), carbohydrates, Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol and L-carnitine.

In a further embodiment, the human milk like product according to the present invention also comprises at least one bioactive selected in the group consisting of: growth factors, cytokines, probiotics, extracellular vesicles (e.g. milk fat globules and or exosomes), bioactives from exosomes (for example miRNA) and secretory IgA.

Such human breast milk like product may be prepared according to the method of the present invention for example by including a step C) of addition of growth factors, cytokines, probiotics, extracellular vesicles (e.g. milk fat globules and or exosomes), bioactives from exosomes (for example miRNA) and secretory IgA.

In one embodiment, the human breast milk like product contains probiotics.

Such human breast milk like product may be prepared according to the method of the present invention for example by including a step C) of addition of probiotics (for example *B.Lactis, B.Infantis, L. Ramnhosus)* which can be obtained from several commercially available sources.

In such embodiment, the human breast milk like product may be used for optimizing gastro intestinal function and/or promoting Immunity.

In one embodiment, the human breast milk like product contains secretory IgA and probiotics.

Such human breast milk like product may be prepared according to the method of the present invention for example by including a step C) of addition of a combination of probiotics and secretory IgA which may be prepared as described for example in patent applications WO2009/156301 and WO2009/156367 which are hereby incorporated by reference. In such embodiment, the human breast milk like product may be used for preventing Immunoglobulin deficiency and/or in the prevention of recurrent infection in infants and young children.

### 'Non-standard' human breast milk like product

In one embodiment of the present invention, the human milk like product can have altered ratios and concentrations of components found naturally in human breast milk of a well-nourished mother. This is referred to herein as a "non-standard milk like product".

In one embodiment, the human milk like product according to the present invention may be selected from the group consisting of a milk fortifier, a supplement, and/or a human breast milk replacer adapted for special purposes.

### Human Milk fortifiers and Human milk bioactive supplements

In one embodiment, the method of the present invention provides for a human breast milk like product which may be used to fortify human breast milk naturally obtained from a nursing mother or to fortify infant formulas.

In another embodiment, the method of the present invention provides for a human breast milk like product which may be used as a supplement for infants or young children in need thereof.

In such embodiments the human breast milk like products may be used for providing healthy growth and/or to reduce the risk of developing a disease typically associated to specific conditions in an infant or young child (such as for example asthma, allergy, cognitive alterations) and /or to promote catch up growth, development of immunity, protection from infections.

Remarkably, the human origin of the constituents (especially bioactive constituents) in such fortifiers or supplements combined with the fact that they are according to the method of the invention, is supposed to provide to such constituents an intact or higher functionality.

The human breast milk like product is preferably intended to be used as a fortifier. Such human breast milk like product intended to be used as a fortifier and may be prepared according to the method of the present invention for example by including a step C) of isolation and/or enrichment of (certain) bioactives from the human breast milk like product obtainable from step B). Such isolation step may be performed via classical fractionation, enrichment and/or purification of the non-modified human breast milk like product obtainable from step B).

The human breast milk like product intended to be used as a supplement may comprise one or more bioactives selected from the group consisting of: human milk oligosaccharides (for example 2FL, 3FL, LNT, LnNT, DiFl, 6SL and /or 3SL), lipids, growth factors (for example epidermal growth factor (EGF), heparin binding epidermal growth factor), cytokines (for example transforming growth factor -beta 2 (TGFbeta-2), IL-1. IL-2, IL-6, IL-10, IL-18, interferon gamma (INF-gamma), TNF-alpha), extracellular vesicles (e.g. milk fat globules and or exosomes), exosome comprising microRNAs and antimicrobial/protecting bioactives (for example IgA, lactoferrin, lysozyme, lactadherine). Such human breast milk like product intended to be used as a supplement may be prepared according to the method of the present invention for example by including a step C) of isolation of the bioactives from the non-modified human breast milk like product obtainable from step B). Such isolation step may be performed via classical fractionation, enrichment and/or purification of the non -modified human breast milk like product obtainable from step B).

In one embodiment, the human breast milk like product is a supplement or milk fortifier which contains fucosylated human milk oligosaccharides, for example 2FL and/or 3FL. Such supplement or milk fortifier is for use in completing the profile of human breast milk of women who do not secrete fucosylated oligosaccharides because of the inactivity of their FUT2 gene.

Such human breast milk like product intended to be used as a fortifier or supplement may be prepared according to the method of the present invention for example by including a step C) of isolation and/or enrichment of fucosylated oligosaccharides (for example 2FL and or 3FL) from the non-modified human breast milk like product obtainable from step B).

In such an embodiment, the human breast milk like product may be used for optimizing gastro intestinal function and/or promoting Immunity.

### Human breast milk like product for infants with genetic diseases

In one embodiment, the human breast milk like product according to the present invention may be adapted to address the specific need of infants who are born with a genetic disease.

### Galactossemia

In such embodiment, the human breast milk like product may be adapted to the needs on infants suffering from Galactossemia. Galactossemia is a rare genetic disease that affects babies' ability to metabolize galactose.

In such embodiment, the human breast milk like product should be deprived of lactose and/or lactose containing saccharides. In such embodiment human breast milk like product may be used for providing healthy growth to the infants affected by galactossemia.

In one embodiment, a human breast milk like product deprived of lactose and/or lactose containing saccharides may be obtained according to the method of the present invention by including a step C) of enzymatic treatment (lactase treatment), or of membrane fractionation and ultrafiltration of the non-modified human breast milk like product obtainable from step B).

In another embodiment, a human breast milk like product deprived of lactose and/or lactose containing saccharides may be obtained according to the method of the present invention by using under step A) GMO alpha-lactalbumin deficient human cells to generate hiPSCs.

### Phenyl Keturonia

In such an embodiment, the human breast milk like product may be adapted to the needs on infants suffering from Phenyl Keturonia (PKU). PKU is due to absent or dysfunctional phenylalanine hydroxylase, which converts phenylalanine to tyrosine. Untreated, it leads to severe mental retardation due to brain toxicity.

In such an embodiment, the human breast milk like product should be deprived or depleted of phenylalanine.

In such an embodiment, the human breast milk like product may be used for providing healthy growth to the infants affected by PKU.

In one embodiment, the human breast milk like product is depleted of phenyl alanine in such a way that phenylalanine content is kept below 20 mg/kg body weight of the subject receiving it.

In one embodiment, a human breast milk like product depleted or deprived of phenylalanine may be obtained according to the method of the present invention by including a step C) of enzymatic treatment (protein hydrolysis) or of filtration of the non -modified human breast milk like product obtainable from step B).

In one embodiment, a human breast milk like product depleted of phenylalanine may be obtained according to the method of the present invention by including a step C) of enzymatic treatment (protein hydrolysis) or of filtration of the non - modified human breast milk like product obtainable from step B).

In another embodiment, a human breast milk like product depleted of phenylalanine may be obtained according to the method of the present invention by providing in step B) a culture medium providing limited or zero amounts of phenylalanine, such as for example a culture medium containing Glycomacropeptide (GMP) from whey.

### Components of human milk like products

As previously described herein, the human milk like product according to the present invention comprises at least one bioactive. One such bioactive is exosomes.

### Exosomes

Human breast milk exosomes are small microvesicles involved in infant development, for example in immunity, metabolism and development. They are thought to play an important role in therapy and disease prevention.

The exosomes comprise a lipid bilayer membrane that encapsulates a combination of molecules including proteins, lipids, messengerRNA (mRNA) and microRNA (miRNA). The types and concentrations of these molecules determine the exosome's fingerprint and function.

The present invention provides isolated human breast milk exosomes. The exosomes of present invention possess the same lipid bilayer vesicle structure and function to that of a naturally derived human breast milk exosome.

The exosomes of the invention are not produced from naturally derived human breast milk.

In some embodiments, the present invention provides exosomes that have been isolated from the human milk like product described anywhere herein, wherein the human milk like product is produced from any method described herein.

Thus, for example, the present invention relates to an *in vitro* method for producing exosomes, the method comprising:
A) Generating lactocyte mammary-like gland organoids derived from human induced pluripotent stem cells (hiPSC)
B) Secreting a human milk like product from said lactocytes, and
C) Purifying the exosomes from the human milk like product by chromatography or filtration or ultracentrifugation in order to isolate the exosomes,

wherein step A) comprises culturing the hiPSCs in a culture medium comprising BMP4 and/or RA,
optionally, wherein step A) is conducted for a total of 42 days or 31 days, and/or
wherein step A) is conducted in 3D-suspension conditions.

The exosomes of the present invention differ from exosomes derived from natural human breast milk by their composition.

In some embodiments, the exosomes of the present invention differ from exosomes derived from natural human breast milk by their proteome profile. It will be understood that a proteome profile relates to the types of proteins present in the exosomes and/or the concentration of the proteins present in the exosomes.

In some embodiments, the exosomes of the present invention are substantially the same as exosomes derived from natural human breast milk by one or more characteristics selected from: size, miRNA profile and/or lipid profile. In some embodiments, the size, miRNA profile and lipid profile of the exosomes of the present invention are substantially the same as exosomes derived from natural human breast milk.

In some embodiments, differences in the proteome profile is the only difference between exosome of the present invention and exosomes derived from natural human breast milk.

The present invention also relates to a population of exosomes as described anywhere herein. In some embodiments, the exosomes in said population are identical or different in terms of their structure, function and/or composition. In some embodiments, the exosomes in said population are identical. In some embodiments, the exosomes in said population differ by their composition.

In some embodiments, the present invention relates to a composition comprising the exosomes or population of exosomes as described anywhere herein. In some embodiments, the present invention relates to a nutritional composition comprising the exosomes or population of exosomes as described anywhere herein.

### Exosome proteomics

Through proteomics analysis, about 2000 proteins have been identified in human breast milk exosomes in the literature.

The inventors of the present invention carried out proteomics analysis of the exosomes of the present invention and surprisingly demonstrated that the exosomes possess similar but not identical proteomes to those of exosomes derived from natural human breast milk.

In particular, exosomes of the present invention were found to be missing one or more proteins that are present in exosomes derived from natural human breast. These proteins include but are not limited to C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine—tRNA ligase 1, cytoplasmic (TARS1), and Cytosolic non-specific dipeptidase (CNDP2).

However, importantly, the exosomes of the present invention were found to comprise proteins known in the literature to be present in exosomes derived from natural human breast milk that are key to infant health and development. These proteins include Lactotransferrin (LTF), Annexin A2 (ANXA2) and Lactadherin (MFGE8). In some embodiments, these proteins include Lactotransferrin (LTF), Annexin A2 (ANXA2) and Lactadherin (MFGE8), MUC1 and tetraspanins CD9, CD81 and CD63.

Thus, in some embodiments, the invention provides a human breast milk exosome, wherein the exosome does not comprise one or more proteins selected from C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine-tRNA ligase 1, cytoplasmic (TARS1), and Cytosolic non-specific dipeptidase (CNDP2).

In some embodiments, the exosome does not comprise C4a anaphylatoxin (CFA).

In some embodiments, the exosome does not comprise Hypoxia up-regulated protein 1 (HYOU1).

In some embodiments, the exosome does not comprise Apolipoprotein A-IV (APOA4).

In some embodiments, the exosome does not comprise Procollagen-lysine,2-oxoglutarate 5-dioxygenase 1 (PLOD1).

In some embodiments, the exosome does not comprise Threonine—tRNA ligase 1 cytoplasmic (TARS1).

In some embodiments, the exosome does not comprise Cytosolic non-specific dipeptidase (CNDP2).

In some embodiments, the exosome does not comprise proteins C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine-tRNA ligase 1, cytoplasmic (TARS1), and Cytosolic non-specific dipeptidase (CNDP2).

In some embodiments, the exosome comprises one or more of the proteins listed in Table 1. In some embodiments, the exosome comprises all of the proteins listed in Table 1.

**Table 1**

| **Description** | **Gene** |
|---|---|
| Fatty acid synthase | FASN |
| Fibronectin | FN1 |
| Xanthine dehydrogenase/oxidase | XDH |
| Ras GTPase-activating-like protein IQGAP1 | IQGAP1 |
| Cytoplasmic dynein 1 heavy chain 1 | DYNC1H1 |
| Programmed cell death 6-interacting protein | PDCD6IP |
| Lactotransferrin (Fragment) | LTF |
| Alpha-actinin-4 | ACTN4 |
| Keratin, type II cytoskeletal 1 | KRT1 |
| Keratin, type II cytoskeletal 2 epidermal | KRT2 |
| Tenascin | TNC |
| Transitional endoplasmic reticulum ATPase | VCP |
| Unconventional myosin-lc | MYO1C |
| Coatomer subunit alpha | COPA |
| ATP-citrate synthase | ACLY |
| Ezrin | EZR |
| Elongation factor 2 | EEF2 |
| Keratin, type I cytoskeletal 9 | KRT9 |
| Glycogen phosphorylase, brain form | PYGB |
| Annexin A2 | ANXA2 |
| Butyrophilin subfamily 1 member A1 | BTN1A1 |
| Galectin-3-binding protein | LGALS3BP |
| Keratin, type I cytoskeletal 10 | KRT10 |
| Apolipoprotein A-I | APOA1 |
| Bile salt-activated lipase | CEL |
| Protein Niban 2 | NIBAN2 |
| Heat shock cognate 71 kDa protein | HSPA8 |
| Lactadherin | MFGE8 |
| Prostaglandin F2 receptor negative regulator | PTGFRN |
| Myoferlin | MYOF |
| Bifunctional glutamate/proline--tRNA ligase | EPRS1 |
| Perilipin-2 | PLIN2 |
| Perilipin-3 | PLIN3 |
| Glutamine--fructose-6-phosphate aminotransferase [isomerizing] 1 | GFPT1 |
| Endoplasmin | HSP90B1 |
| Actin-depolymerizing factor | GSN |
| 4F2 cell-surface antigen heavy chain | SLC3A2 |
| Endoplasmic reticulum chaperone BiP | HSPA5 |
| Heat shock protein HSP 90-beta | HSP90AB1 |
| Clusterin | CLU |
| Moesin | MSN |
| Heat shock 70 kDa protein 1B | HSPA1B |
| Phosphoglycerate kinase 1 | PGK1 |
| Aminopeptidase N | ANPEP |

In some embodiments, the exosome comprises Lactotransferrin (LTF), Annexin A2 (ANXA2) and/or Lactadherin (MFGE8). In some embodiments, the exosome comprises Lactotransferrin (LTF), Annexin A2 (ANXA2) and Lactadherin (MFGE8). In some embodiments, the exosome comprises Lactotransferrin (LTF), Annexin A2 (ANXA2) and Lactadherin (MFGE8), MUC1 and tetraspanins CD9, CD81 and CD63.

In some embodiments, the exosome comprises said one or more proteins described anywhere herein in substantially the same concentration as the same one or more proteins in exosomes derived from natural human breast milk.

In some embodiments, the exosome comprises said one or more proteins described anywhere herein in different concentrations as the same one or more proteins in exosomes derived from natural human breast milk. In some embodiments, said one or more proteins may be present in a higher concentration compared to the concentration of said one or more proteins in exosomes derived from natural human breast milk. In some embodiments, said one or more proteins may be present in a lower concentration compared to the concentration of said one or more proteins in exosomes derived from natural human breast milk.

As described herein, in some embodiments, the present invention relates to a composition comprising the exosomes or population of exosomes as described anywhere herein. In some further embodiments, the present invention relates to a composition comprising i) the exosomes or population of exosomes as described anywhere herein and ii) one or more proteins that are absent from the exosomes of the present invention but are present in exosomes derived from natural human breast milk. Said proteins may be selected from but not limited to one or more of proteins C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine—tRNA ligase 1, cytoplasmic (TARS1), and Cytosolic non-specific dipeptidase (CNDP2).

### Exosome miRNA profile

The inventors of the present invention carried out miRNA analysis of the exosomes of the present invention and surprisingly demonstrated that the exosomes possess substantially the same miRNA profile to that of exosomes derived from natural human breast milk.

In particular, exosomes of the present invention were surprisingly found to comprise miRNAs known in the literature to be present in exosomes derived from natural human breast milk that are key to infant health and development. These miRNAs include miR-148a-3p, miR-22-3p, miR-125b-5b and miR-6126.

Thus, in some embodiments, the invention provides a human breast milk exosome, wherein the exosome comprises one or more miRNAs selected from miR-148a-3p, miR-22-3p, miR-125b-5b and miR-6126.

In some embodiments, the exosome comprises miR-148a-3p, miR-22-3p, miR-125b-5b and miR-6126.

In some embodiments, the exosome comprises said one or more miRNAs described anywhere herein in substantially the same concentration as the same one or more miRNAs in exosomes derived from natural human breast milk.

In some embodiments, the exosome comprises said one or more miRNAs described anywhere herein in different concentrations as the same one or more miRNAs in exosomes derived from natural human breast milk. In some embodiments, said one or more miRNAs may be present in a higher concentration compared to the concentration of said one or more miRNAs in exosomes derived from natural human breast milk. In some embodiments, said one or more miRNAs may be present in a lower concentration compared to the concentration of said one or more miRNAs in exosomes derived from natural human breast milk.

### Exosome lipidomics

The inventors of the present invention carried out lipid analysis of the exosomes of the present invention and surprisingly demonstrated that the exosomes possess substantially the same lipid profile to that of exosomes derived from natural human breast milk.

In particular, exosomes of the present invention were surprisingly found to comprise lipid classes known in the literature to be present in exosomes derived from natural human breast milk that are key to infant health and development. These lipids include cholesterol esters, ceramides, triacylglycerides, diacylglycerides, lyso-phospholipids, total phospholipids, alkanyl phospholipids, and alkenyl phospholipids.

Of these key lipid classes, phospholipids in particular are considered to be key for exosome structure and function. Exosomes of the present invention were also surprisingly found to comprise phospholipid sub-classes known in the literature to be present in exosomes derived from natural human breast milk. These phospholipids include sphingomyelins, phosphatidylcholine, phosphatidylethanolamine, phosphatidylinositol, and phosphatidylserine.

Thus, in some embodiments, the invention provides a human breast milk exosome, wherein the exosome comprises one or more lipids selected from cholesterol esters, ceramides, triacylglycerides, diacylglycerides, total phospholipids, alkanyl phospholipids, and alkenyl phospholipids.

In some embodiments, the exosome comprises cholesterol esters, ceramides, triacylglycerides, diacylglycerides, total phospholipids, alkanyl phospholipids, and alkenyl phospholipids.

In some embodiments, the exosome comprises one or more phospholipids selected from sphingomyelins, phosphatidylcholine, phosphatidylethanolamine, phosphatidylinositol, and phosphatidylserine.

In some embodiments, the exosome comprises sphingomyelins, phosphatidylcholine, phosphatidylethanolamine, phosphatidylinositol, and phosphatidylserine.

In some embodiments, the exosome comprises said one or more lipids described anywhere herein in substantially the same concentration as the same one or more lipids in exosomes derived from natural human breast milk.

In some embodiments, the exosome comprises said one or more lipids described anywhere herein in different concentrations as the same one or more lipids in exosomes derived from natural human breast milk. In some embodiments, said one or more lipids may be present in a higher concentration compared to the concentration of said one or more lipids in exosomes derived from natural human breast milk. In some embodiments, said one or more lipids may be present in a lower concentration compared to the concentration of said one or more lipids in exosomes derived from natural human breast milk.

### Exosome size

The inventors of the present invention carried out size analysis of the exosomes of the present invention and surprisingly demonstrated that the exosomes are substantially the same size in diameter as exosomes derived from natural human breast milk.

Thus, in some embodiments, the invention provides a human breast milk exosome, wherein the exosome has a diameter size of 50 to 100nm, preferably 60-80nm, more preferably 65 to 75nm.

In some embodiments, the exosome has a diameter size of 65 to 75nm.

It will be understood that any features of the exosomes described anywhere herein may be combined. For example, features relating to the proteome of the exosome may be combine with features relating to the miRNA profile, lipid profile and/or size profile.

### Additional Embodiments of the Invention

The present invention may be described according to the following numbered statements:
S1. An isolated human breast milk exosome, wherein the exosome does not comprise one or more proteins selected from C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine—tRNA ligase 1, cytoplasmic (TARS1), and Cytosolic non-specific dipeptidase (CNDP2).
S2. The exosome of statement 1, wherein the exosome comprises lactotransferrin (LTF), annexin A2 (ANXA2) and lactadherin (MFGE8), MUC1 and tetraspanins CD9, CD81 and CD63.
S3. The exosome of statement 1 or 2, wherein the exosome comprises one or more miRNAs selected from miR-148a-3p, miR-22-3p, miR-125b-5b and miR-6126.
S4. The exosome of any preceding statement, wherein the exosome comprises one or more lipids selected from cholesterol esters, ceramides, triacylglycerides, diacylglycerides, lyso-phospholipids, total phospholipids, alkanyl phospholipids, and alkenyl phospholipids.
S5. The exosome of any preceding statement, wherein the exosome comprises one or more phospholipids selected from sphingomyelins, phosphatidylcholine, phosphatidylethanolamine, phosphatidylinositol, and phosphatidylserine.
S6. The exosome of any preceding statement, wherein the exosome has a diameter of 50 to 100nm.
S7. The exosome of statement 6, wherein the exosome has a diameter size of 65 to 75nm.
S8. The exosome of any preceding statement, wherein the exosome has the same function as exosomes derived from natural human breast milk.
S9. The exosome of any preceding statement, wherein the exosome has the same structure as exosomes derived from natural human breast milk.
S10. A population of exosomes, wherein the population comprises exosomes of any preceding statement.
511. A composition comprising exosomes or a population of exosomes according to any preceding statement.
S12. A nutritional composition comprising exosomes or a population of exosomes according to any one of statements 1 to 10.
S13. An *in vitro* method for producing mammalian breast milk exosomes, the method comprising:
   A) Generating lactocyte mammary-like gland organoids derived from mammalian induced pluripotent stem cells (miPSC)
   B) Secreting a mammalian milk like product from said lactocytes, and
   C) Purifying the exosomes from the mammalian milk like product to remove impurities, optionally by chromatography or filtration or ultracentrifugation, in order to isolate the exosomes,
   wherein step A) comprises culturing the miPSCs in a culture medium comprising BMP4 and/or RA.
S14. The method of statement 13, wherein step A) comprises:
   i) culturing the mammalian induced pluripotent stem cells (miPSCs) in a culture medium comprising BMP4 and/or RA to generate embryoid bodies (EBs), and
   ii) growing the EBs to generate a population of mammary cells.
S15. The method of statement 14, wherein the culturing step i) comprises culturing the miPSCs in MammoCult medium and BMP4, in a 3D-suspension culture system, for example 3D-suspension condition, thereby directing the iPSCs to differentiate towards non-neural ectoderm cells, optionally for at least 12 days.
S16. The method of statement 14, wherein the culturing step i) comprises culturing the miPSCs in MammoCult medium and BMP4, in a 3D-suspension culture system, for example 3D-suspension condition, thereby directing the iPSCs to differentiate towards non-neural ectoderm cells, optionally for no more than 8 days.
S17. The method of statements 14 or 15, wherein the growing step ii) comprises growing the formed EBs in a 3D embedding system comprising RA, for example a mixed floating gel composed of matrix protein such as Matrigel and/or Collagen I for at least 30 days, for example for 32 days, to generate lactocytes.
S18. The method of statements 14 or 16, wherein the growing step ii) comprises growing the formed EBs in a 3D embedding system comprising RA, for example a mixed floating gel composed of matrix protein such as Matrigel and/or Collagen I for no more than 25 days, to generate lactocytes.
S19. The method of statement 17, wherein step ii) is distinguished into further substeps and comprises the following steps: ii) and iii):
   ii) incubation of mEBs (mammospheres) in complete EpiCultB medium supplemented with EpiCult proliferation supplement, Parathyroid hormone (pTHrP), and RA for 5 days,
   iii) promotion of branch and alveolar differentiation and mammary cell specification by incubating mEBs (mammospheres) in EpiCultB medium supplemented with EpiCult proliferation supplement, hydrocortisone, insulin, FGF10 and HGF for 20 days.
S20. The method of statement 18, wherein step ii) is distinguished into further substeps and comprises the following steps: ii) and iii):
   ii) embedding the formed mEBs (mammospheres) in a mixture of Matrigel and Collagen I floated in EpiCultB medium supplemented with EpiCult proliferation supplement, Parathyroid hormone (pTHrP), and RA for 5 days,
   iii) promotion of branch and alveolar differentiation and mammary cell specification by incubating embedded mEBs (mammospheres) in EpiCultB medium supplemented with EpiCult proliferation supplement, hydrocortisone, insulin, FGF10 and HGF for 15 days.
S21. The method of any one of statements 13 to 20, wherein the mammary gland cells are human mammary gland cells.
S22. The method of any one of statements 13 to 21, wherein BMP4 is added to the culture medium between day 0 and day 10, preferably between day 0 and day 3, where day 0 is the time point where the iPSCs are first added to the culture medium.
S23. The method of any one of statements 1 to 22, wherein BMP4 is added to the culture medium for 3 days.
S24. The method of any one of statements 1 to 23, wherein RA is added to the culture medium between day 10 and day 15, optionally between day 6 and day 11, where day 0 is the time point where the iPSCs are first added to the culture medium.
S25. The method of any one of statements 1 to 24, wherein RA is added to the culture medium for 5 days.
S26. The method of any one of statements 1 to 25, wherein BMP4 is added to the culture medium in a concentration of 5 to 20 ng/mL, preferably 5 ng/mL, 10 ng/m L or 20 ng/mL.
S27. The method of any one of statements 1 to 26, wherein RA is added to the culture medium in a concentration of 1 µM.
S28. The method of any one of statements 1 to 27, wherein the EBs express one or more mammary gland positive progenitor-cell markers, optionally selected from EpCAM, CD49f, MUC1 and GATA3.
S29. The method of any one of statements 1 to 28, wherein the EBs have increased expression of one or more mammary gland positive progenitor-cell markers compared to the expression level of said mammary gland positive progenitor-cell markers in EBs not treated with BMP4 and RA.
S30. The method of any one of statements 1 to 29, wherein at least 10%, 20%, 30%, 40%, 50%, 60%, 70%, 80% or more of the EBs express one or more mammary gland positive progenitor-cell markers, optionally wherein at least 35% of EBs express EpCAM and CD49f mammary gland positive progenitor-cell markers, optionally wherein at least 15% of EBs express MUC1 and EpCAM mammary gland positive progenitor-cell markers, and/or optionally wherein at least 20% of EBs express EpCAM and GATA3 mammary gland positive progenitor-cell markers.
S31. The method of any one of statements 1 to 30, wherein the EBs express one or more milk-specific bioactive markers, optionally osteopontin (OPN).
S32. The method of any one of statements 1 to 31, wherein the EBs have increased expression one or more milk-specific bioactive markers, optionally increased expression of osteopontin (OPN).
S33. The method of any one of statements 1 to 32, wherein the EBs have increased secretion one or more milk-specific bioactive markers, optionally increased secretion of osteopontin (OPN).
S34. The method of any preceding statement, wherein step A) is between a 30-day and 45-day process, optionally less than a 45-day process, optionally less than a 44-day process, optionally less than a 35-day process, optionally less than a 31-day process.
S35. The method of any preceding statement, wherein the method is for producing a human milk like product, wherein step A) further comprises:
   i) directing hiPSCs to differentiate towards non-neural ectoderm cells by culturing them in an appropriate culture medium comprising BMP4, for example MammoCult medium and BMP4, in an appropriate 3D culture system, for example 3D-suspension condition, for at least 12 days and
   ii) growing the formed mEBs (mammospheres) in an appropriate 3D embedding system comprising RA, for example a mixed floating gel composed of matrix protein such as Matrigel and/or Collagen I and RA for at least 30 days, for example for 32 days, to generate lactocytes.
S36. The method of any preceding statement, wherein the method is for producing a human milk substitute product,
   wherein step A) further comprises:
   i) directing hiPSCs to differentiate towards non-neural ectoderm cells by culturing them in an appropriate culture medium comprising BMP4, for example MammoCult medium and BMP4, in an appropriate 3D culture system, for example 3D-suspension condition, for no more than 8 days and
   ii) growing the formed mEBs (mammospheres) in an appropriate 3D embedding system comprising RA, for example a mixed floating gel composed of matrix protein such as Matrigel and/or Collagen I and RA for no more than 25 days, to generate lactocytes.
S37. The method according to statement 35 wherein step A)i) is defined as follows:
   i) generation of embryoid bodies (EBs) from hiPSCs by incubation in standard iPSC medium E8 comprising DMEM/F12, L-ascorbic acid-2-phosphate magnesium, sodium selenium, FGF2, insulin, NaHCO₃ and transferrin, TGFβ1 or NODAL or in medium mTeSR^{™} for two days, and producing mEBs (mammospheres) highly enriched in non-neural ectodermal cells by incubation of EBs in complete MammoCult medium comprising the basal medium, proliferation supplement and supplemented with BMP4, heparin, and hydrocortisone for 10 days, and wherein step A)ii) is distinguished into further substeps and comprises the following steps: ii), iii) and iv):
   ii) incubation of mEBs (mammospheres) in complete EpiCultB medium supplemented with EpiCult proliferation supplement and Parathyroid hormone (pTHrP) and RA for 5 days,
   iii) promotion of branch and alveolar differentiation and mammary cell specification by incubating mEBs (mammospheres) in EpiCultB medium supplemented with EpiCult proliferation supplement, hydrocortisone, insulin, FGF10 and HGF for 20 days, and
   iv) induction of milk protein expression by incubating mEBs (mammospheres) in EpiCultB medium supplemented with EpiCult proliferation supplement, hydrocortisone, insulin, FBS, prolactin, progesterone and β-estradiol for 7 days.
S38. The method according to statement 36 wherein step A)i) is defined as follows: i) generation of embryoid bodies (EBs) from hiPSCs by incubation in standard iPSC medium E8 comprising DMEM/F12, L-ascorbic acid-2-phosphate magnesium, sodium selenium, FGF2, insulin, NaHCO₃ and transferrin, TGFβ1 or NODAL or in medium mTeSR^{™} for two days, and producing mEBs (mammospheres) highly enriched in non-neural ectodermal cells by incubation of EBs in complete MammoCult medium comprising the basal medium, proliferation supplement and supplemented with BMP4, heparin, and hydrocortisone for 6 days, and wherein step A)ii) is distinguished into further substeps and comprises the following steps: ii), iii) and iv):
   ii) incubation of mEBs (mammospheres) in complete EpiCultB medium supplemented with EpiCult proliferation supplement and Parathyroid hormone (pTHrP) and RA for 5 days,
   iii) promotion of branch and alveolar differentiation and mammary cell specification by incubating mEBs (mammospheres) in EpiCultB medium supplemented with EpiCult proliferation supplement, hydrocortisone, insulin, FGF10 and HGF for 15 days, and
   iv) induction of milk protein expression by incubating mEBs (mammospheres) in EpiCultB medium supplemented with EpiCult proliferation supplement, hydrocortisone, insulin, FBS, prolactin, progesterone and β-estradiol for 5 days.
S39. The method according to statement 35 wherein step A)i) is defined as follows: i) generation of embryoid bodies (EBs) from hiPSCs by incubation in standard iPSC medium E8 comprising DMEM/F12, L-ascorbic acid-2-phosphate magnesium, sodium selenium, FGF2, insulin, NaHCO₃ and transferrin, TGFβ1 or NODAL for two days, and producing mEBs (mammospheres) highly enriched in non-neural ectodermal cells by incubation of EBs in MammoCultB medium supplemented with MammoCult proliferation supplement, hydrocortisone, heparin and BMP4 for 10 days, and wherein step A)ii) is distinguished into further substeps and comprises the following steps ii), iii) and iv):
   ii) embedding the formed mEBs (mammospheres) in a mixture of Matrigel and Collagen I floated in EpiCultB medium supplemented with EpiCult proliferation supplement and Parathyroid hormone (pTHrP) and RA for 5 days,
   iii) promotion of branch and alveolar differentiation and mammary cell specification by incubating embedded mEBs (mammospheres) in EpiCultB medium supplemented with EpiCult proliferation supplement, hydrocortisone, insulin, FGF10 and HGF for 20 days, and
   iv) induction of milk protein expression by incubating mEBs (mammospheres) in EpiCultB medium supplemented with EpiCult proliferation supplement, hydrocortisone, insulin, FBS, prolactin, progesterone and β-estradiol for 7 days.
S40. The method according to statement 36 wherein step A)i) is defined as follows: i) generation of embryoid bodies (EBs) from hiPSCs by incubation in standard iPSC medium E8 comprising DMEM/F12, L-ascorbic acid-2-phosphate magnesium, sodium selenium, FGF2, insulin, NaHCO₃ and transferrin, TGFβ1 or NODAL for two days, and producing mEBs (mammospheres) highly enriched in non-neural ectodermal cells by incubation of EBs in MammoCultB medium supplemented with MammoCult proliferation supplement, hydrocortisone, heparin and BMP4 for 6 days, and wherein step A)ii) is distinguished into further substeps and comprises the following steps ii), iii) and iv):
   ii) embedding the formed mEBs (mammospheres) in a mixture of Matrigel and Collagen I floated in EpiCultB medium supplemented with EpiCult proliferation supplement and Parathyroid hormone (pTHrP) and RA for 5 days,
   iii) promotion of branch and alveolar differentiation and mammary cell specification by incubating embedded mEBs (mammospheres) in EpiCultB medium supplemented with EpiCult proliferation supplement, hydrocortisone, insulin, FGF10 and HGF for 15 days, and
   iv) induction of milk protein expression by incubating mEBs (mammospheres) in EpiCultB medium supplemented with EpiCult proliferation supplement, hydrocortisone, insulin, FBS, prolactin, progesterone and β-estradiol for 5 days.
S41. The method of any preceding statement, wherein Step A) is conducted in 3D suspension culture conditions.
S42. The method of any preceding statement, wherein step C) further comprises formulating the isolated exosomes into a powder form, optionally by spray drying or freeze drying, or liquid form.
S43. The method of any preceding statement, wherein step C) further comprises sterilising the isolated exosomes.
S44. The method of any preceding statement, wherein step C) further comprises storing the isolated exosomes at below freezing, optionally at -80°C.
S45. The method of any preceding statement, further comprising formulating the isolated exosomes with supplementary nutritional ingredients.
S46. The method of any preceding statement, wherein the isolated exosomes are dispensed into a container for consumption.
S47. A mammalian breast milk exosome product which is obtainable according to the method of any one of statements 13 to 46, optionally a human breast milk exosome.
S48. A method of producing a mammalian milk fortifier, comprising conducting the method of any one of statements 13 to 46.

It should be appreciated that the various aspects and embodiments of the detailed description as disclosed herein are illustrative of the specific ways to make and use the invention and do not limit the scope of invention when taken into consideration with the claims and the detailed description. It will also be appreciated that features from aspects and embodiments of the invention may be combined with further features from the same or different aspects and embodiments of the invention.

As used in this detailed description and the appended claims, the singular forms "a," "an" and "the" include plural referents unless the context clearly dictates otherwise.

### Figures

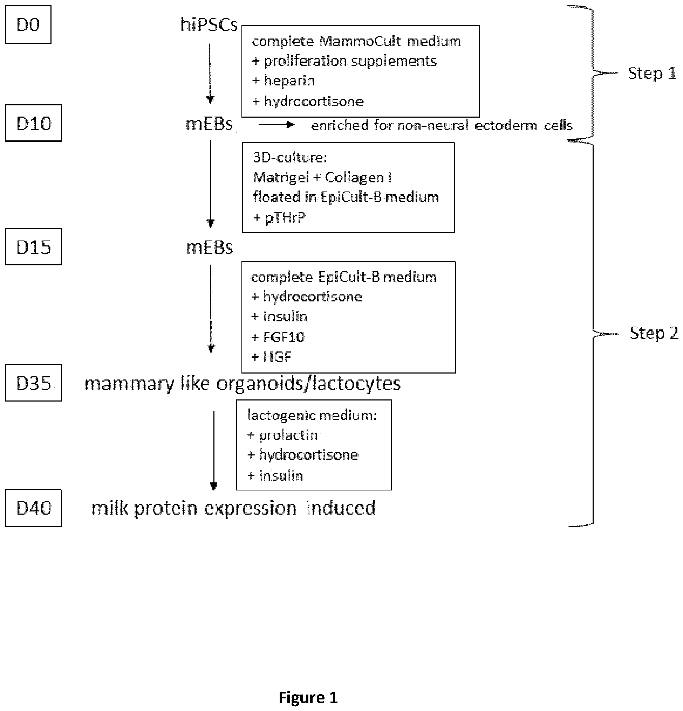

- **Fig. 1:**: shows the differentiation of human induced pluripotent stem cells (hiPSCs) according to the protocol outlined in Ying Qu and as applied in one alternative in step A) of the inventive methods.

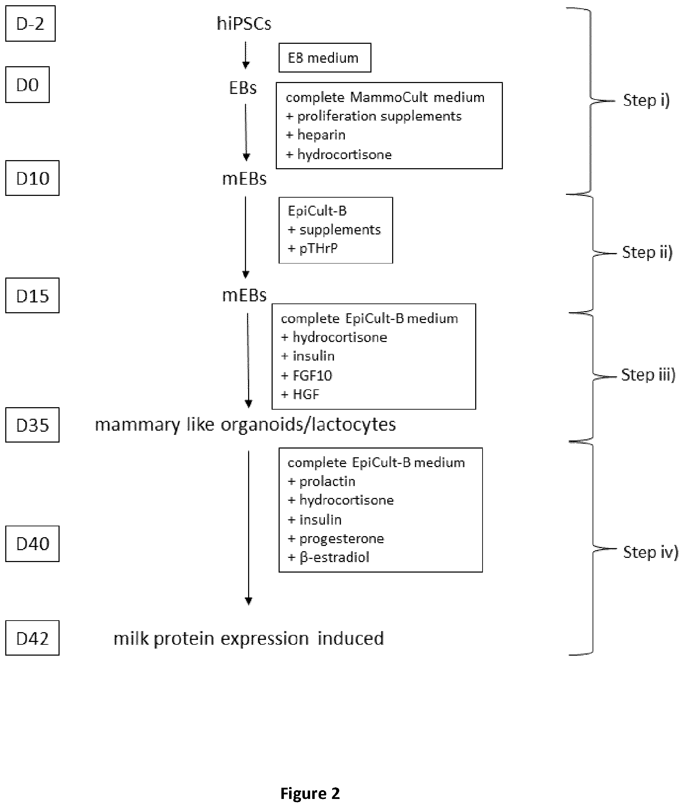

- **Fig. 2:**: shows the differentiation of human induced pluripotent stem cells (hiPSCs) according to step A).

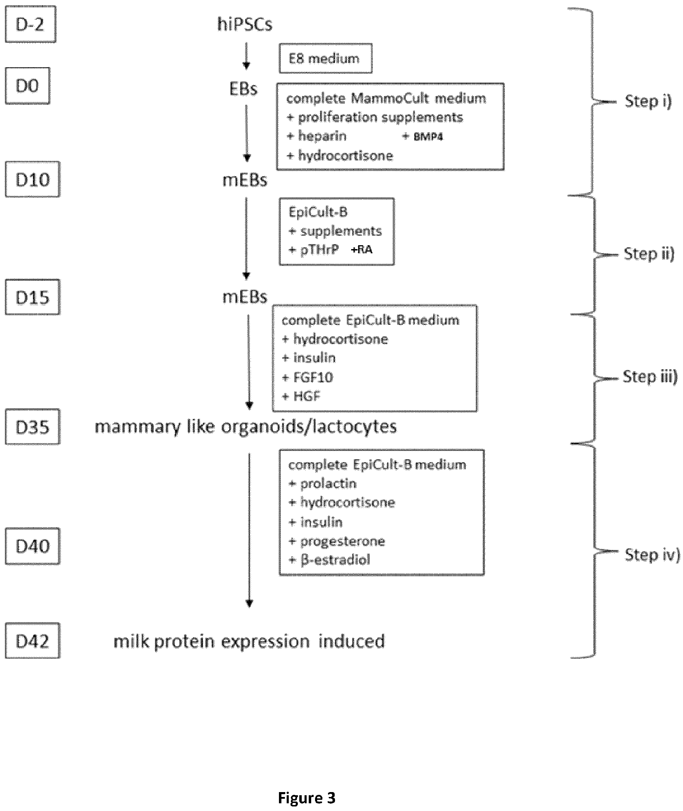

- **Fig. 3:**: shows the differentiation of human induced pluripotent stem cells (hiPSCs) according to the preferred and particularly preferred embodiments for step A) of the inventive methods.

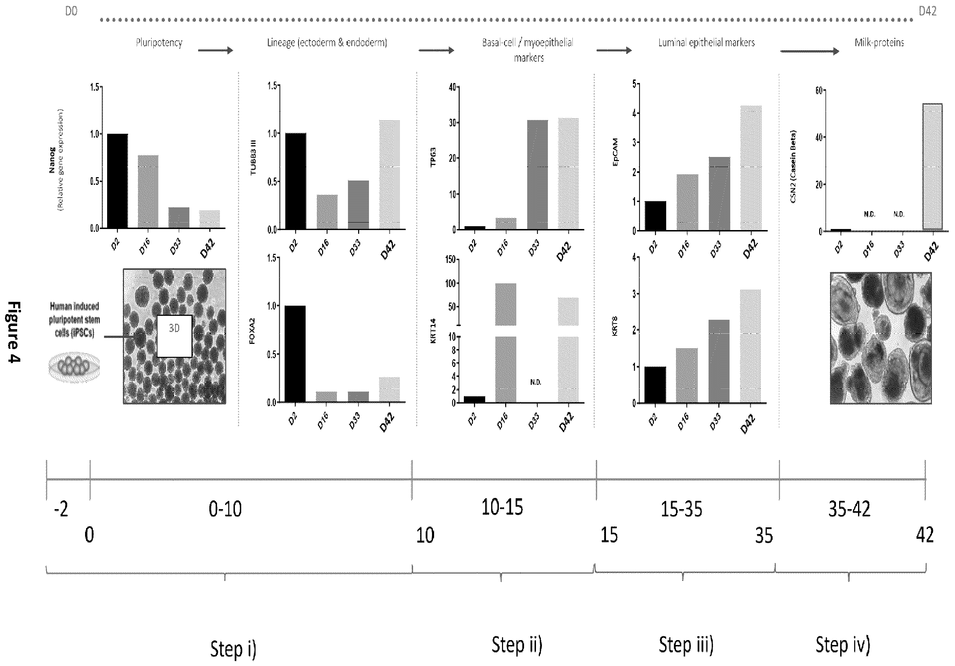

- **Fig. 4:**: shows that three-dimensional organotypic cultures of hiPSCs as produced according to the methods of **Fig. 2** are highly permissive for mammary glands specification. mRNA expression of Nanog, TUBB3, FOXA2, TP63, KR-14, EpCAM, KRT8 and CSN2 for 3D-differentiation (42 days) protocol are shown. Markers from left to right: Stages of Pluripotency (Nanog), Lineage (ectoderm & endoderm) (TUBB3, FOXA2), Basal-cell/myoepithelial markers (TP63, KR-14), Luminal epithelial markers (EpCAM, KRT8), and milk proteins (CSN2 (Casein Beta)).

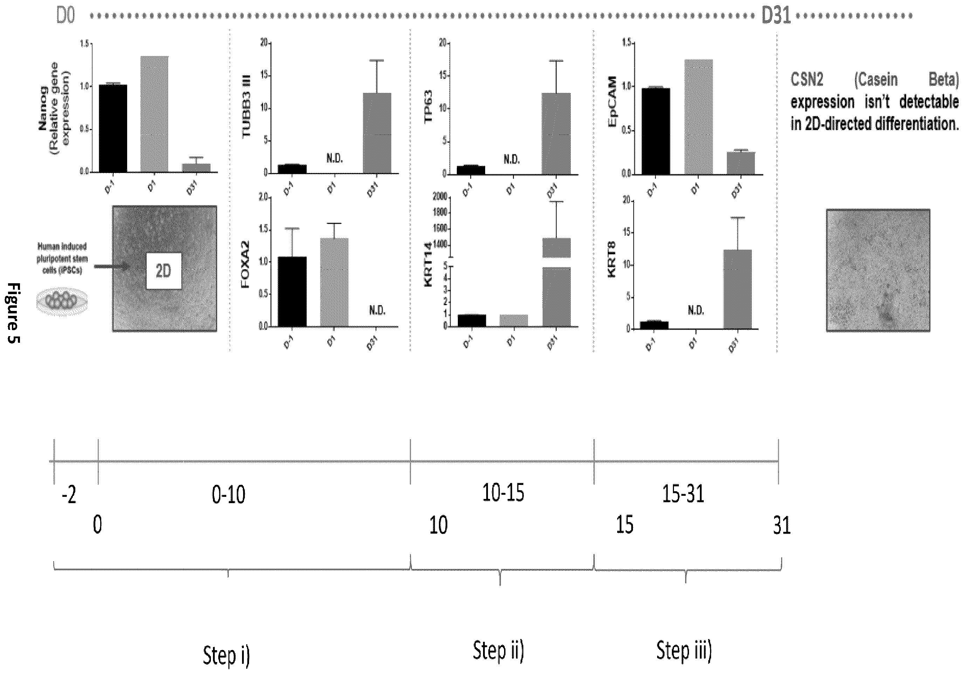

- **Fig. 5:**: shows the two-dimensional organotypic culture of hiPSCs produced as a comparative example. mRNA expression of Nanog, TUBB3, FOXA2, TP63, KR-14, EpCAM, KRT8 and CSN2 for 2D-differentiation (31 days) protocols are shown. Markers from left to right: Sages of Pluripotency (Nanog), Lineage (ectoderm & endoderm) (TUBB3, FOXA2), Basal-cell/myoepithelial markers (TP63, KR-14), Luminal epithelial markers (EpCAM, KRT8), and milk proteins (CSN2 (Casein Beta)).

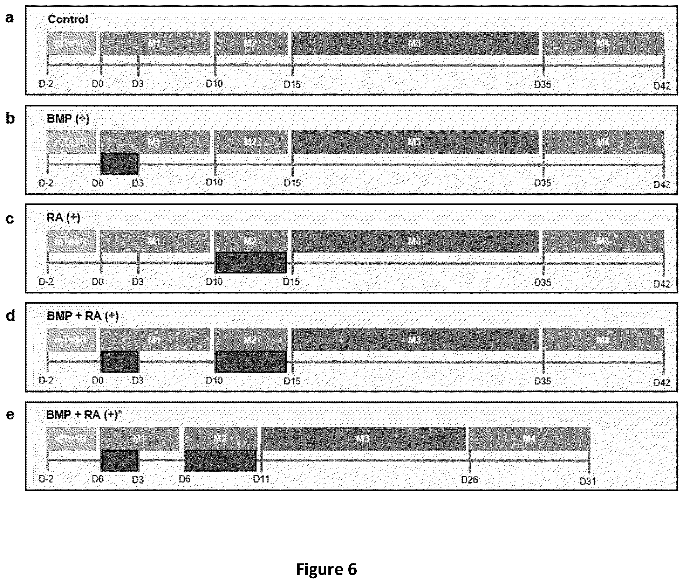

- **Fig. 6:**: shows a schematic illustrations of different mammary-gland differentiation protocols. Scheme summarizing different procedures for the generation of mammary gland progenitors in 42 days (a), using bone morphogenetic protein 4 (BMP4) (b), retinoic acid (RA) (c), combinations of BMP4 and RA (d) and their combination in shortened time-course (31 days) (e). The red boxes indicate the period of addition of BMP4 and/or RA.

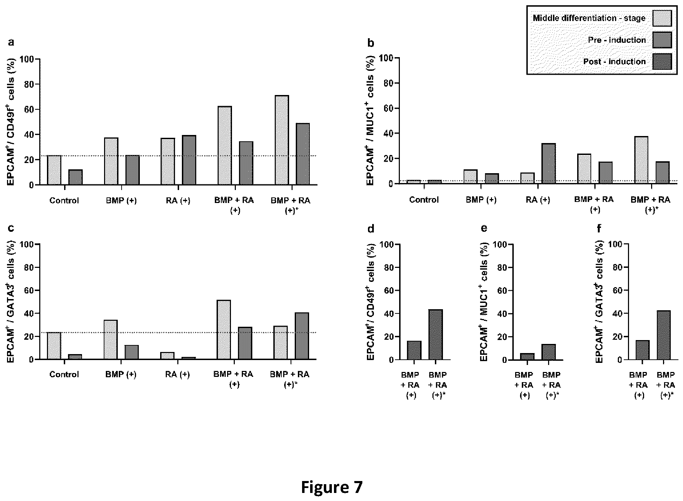

- **Fig 7:**: shows combinatorial effects of bone morphogenetic protein 4 (BMP4) and retinoic acid (RA) on mammary-gland differentiation of induced pluripotent stem cells (iPSCs) using a 3D-organoid model. **a-c**, Flow cytometry quantification of the number of mammary gland positive progenitor-cells using several identified markers: EpCAM, CD49f, MUC1 and GATA3 during the differentiation time-course (day 25 (for control protocols) and day 20 (for shortened time-course protocol)) and pre-induction stages (day 35 (for control protocols including BMP4 and RA), and day 26 (for shortened time-course protocol)) and **d-f,** post-induction period (days 42 (for control protocols including BMP4 and RA) and 31 (for shortened time-course protocol)). ^{∗} *describes shortened-modified protocols (31 days) including BMP4 and RA combinations.*

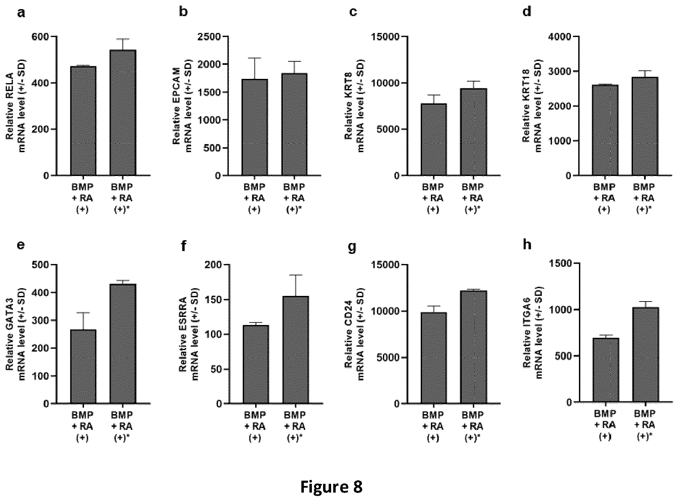

- **Fig 8:**: shows expression of different mammary epithelium markers in lactocytes derived from iPSCs in normal length and shortened protocols **(a-h).**

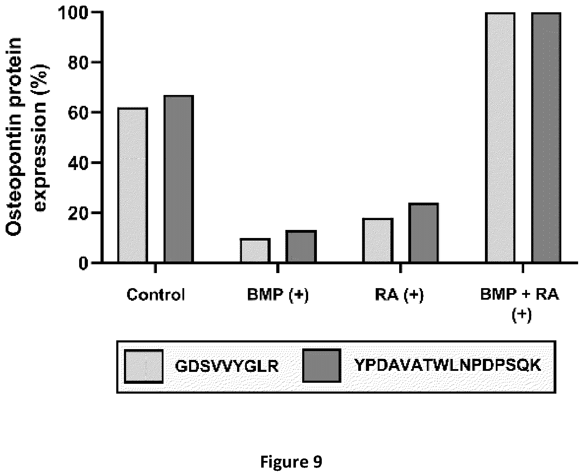

- **Fig 9:**: shows Liquid chromatography-mass spectrometry (LC-MS/MS) based targeted proteomic analysis of osteopontin protein secretion. Osteopontin protein secretion was detected in the media of cultures of iPS-derived mammary-gland cells using control differentiation method, or with bone morphogenetic protein 4 (BMP4), retinoic acid (RA) and in the combination of the two factors.

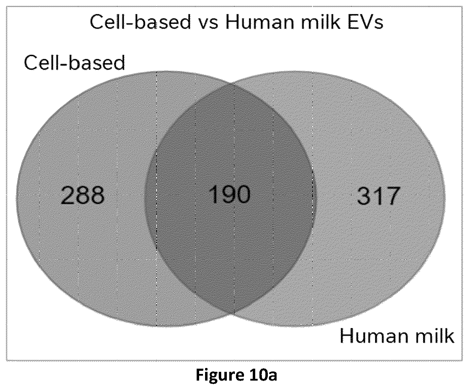

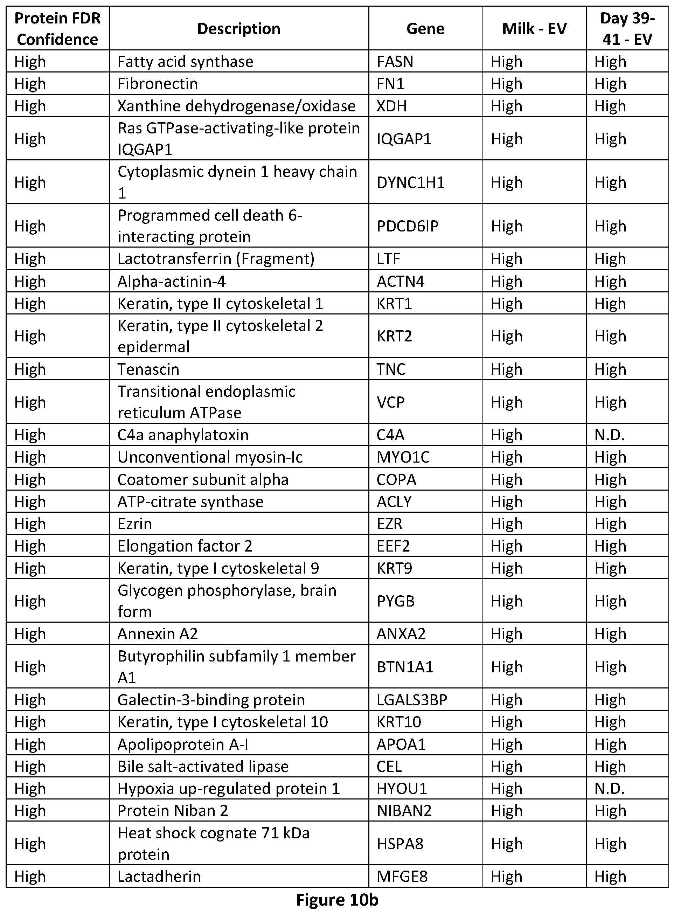

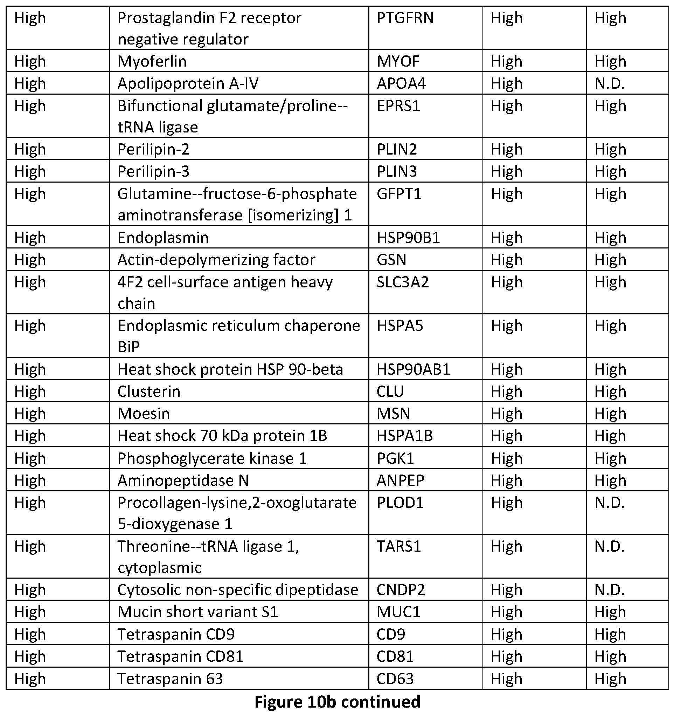

- **Fig 10:**: **a,** Venn diagram shows the number of proteins identified in EVs purified from conditioned media (Day 39-41) only (blue circle), in EVs purified from human milk only (red circle), and in both types of EVs (overlapping blue and red circles); **b,** Table shows the list of the top 50 proteins (based on FDR) detected in both types of EVs (human milk and conditioned media).

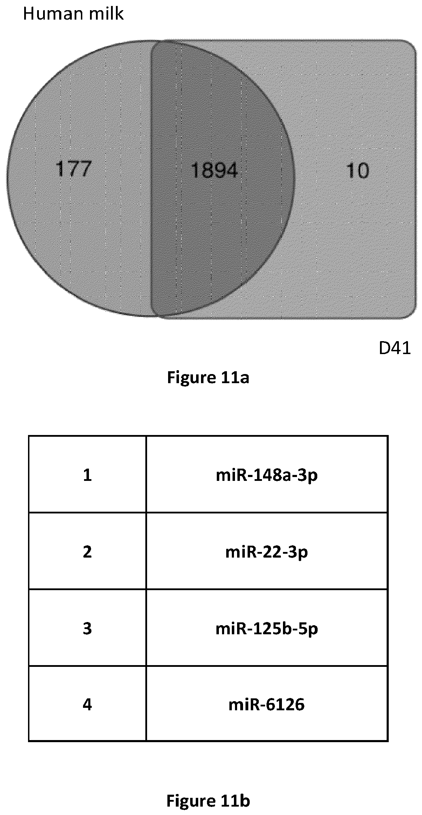

- **Fig 11:**: **a,** Venn diagram shows the number of miRNAs significantly downregulated EVs purified from conditioned media (Day 39-41) compared to human milk EVs (blue circle), significantly upregulated in conditioned media EVs compared to human milk EVs (red square), and not differentially expressed in both types of EVs (overlapping blue and red shapes). **b,** Table shows the list of the top 4 miRNAs (based on FDR) expressed in both types of EVs (human milk and conditioned media).

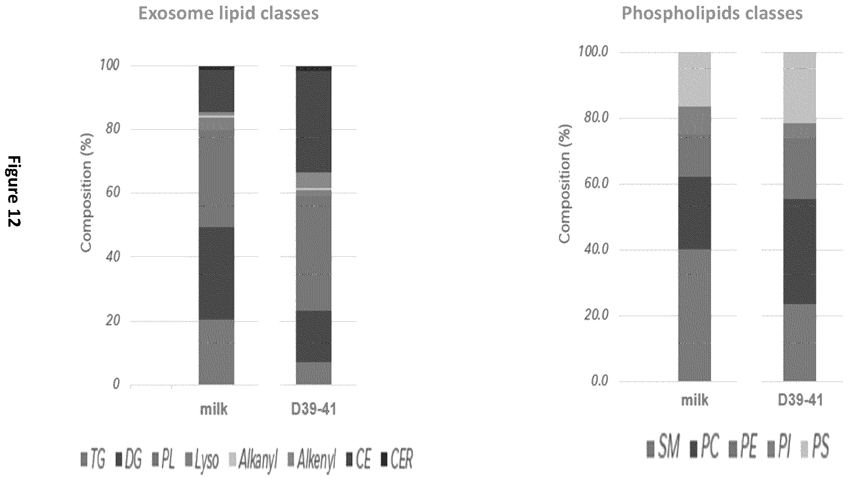

- **Fig 12:**: shows the proportions of lipid (left panel) and phospholipid (right panel) classes found in EVs purified from human milk and conditioned media (Day 39-41). Data is expressed a percentage of total lipid and phospholipid, respectively. TG: Triacylglycerides; DG: Diacylglycerides; PL: Total phospholipids; Lyso: Lyso-phospholipid; Alkanyl: Phospholipid-O; Alkenyl: Phospholipid-P; CE: Cholesterol esters; CER: Ceramides; SM: Sphingomyelins; PC: Phosphatidylcholine; PE: Phosphatidylethanolamine; PI: Phosphatidylinositol; PS: Phosphatidylserine. The specific lipids and phospholipids in the bar charts, starting from the bottom to the top of the charts, correspond to the lipids or phospholipids in the abbreviated lists, starting left to right.

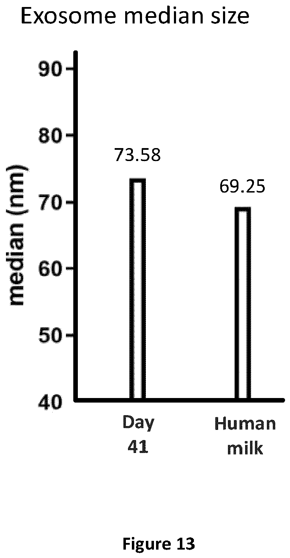

- **Fig 13:**: shows the median particle size (nm) of EVs purified from conditioned media (Day 41) and human milk, analysed my Nano Flow Cytometry.

### Experimental section

### Example 1

### Cultivation and differentiation of hiPSCs into lactocytes to obtain a human milk like product

Lactocytes are cultured starting from ihPSCs according to the procedure described in Ying Qu et al, Stem Cell Report vol 8, 205-215 February 14th 2017 and the human milk like product thereby secreted is collected and can be used in therapy and/or as a breastfeeding substitute according to the present invention.

### Example 2

### Cultivation and differentiation of hiPSCs into 3D-lactocytes to obtain a human milk like product

Lactocytes are cultured starting from hiPSCs according to the method of the present invention following steps A) and B) as described above) and the human milk like product thereby secreted is collected and can be used in therapy and/or as a breastfeeding substitute according to the present invention.

### Example 3

### Alternative methods of cultivation and differentiation of hiPSCs into lactocytes to obtain a human milk like product

Efficient lactocytes differentiation from hiPSCs can be obtained from alternative culture conditions including conditions 1 to 4 as below described:
1. 2D culture on vitronectin coated plates as monolayer of cells derived from the EBs and cultured for at least 28 days in a medium containing (RPMI 1640 with L-glutamine; Fetal bovine serum (FBS); Insulin; Epidermal growth factor (EGF); hydrocortisone; Pen-Strep (penicillin/streptomycin : antibiotic-antimycotic solution).
2. 2D culture on vitronectin coated plates of attached aggregates (EBs) of cells derived from the EBs and cultured for at least 28 days in a medium containing (RPMI 1640 with L-glutamine; Fetal bovine serum (FBS); Insulin; Epidermal growth factor (EGF); hydrocortisone; Pen-Strep (antibiotic-antimycotic solution).
3. 3D culture in suspension in MammoCult medium for at least 10 days and then culture in mixed floating gels (for example Matrigel and Collagen 1) for another 5 days in a specific medium (for example EpiCultB) in presence of Parathyroid hormone followed by 25 days in presence of insulin, HGF, hydrocortisone and FGF10;
4. 3D culture of EBs in suspension (ultra low adherent plate) in MammoCult medium for at least 10 days and then in suspension culture for another 5 days in a specific medium (for example EpiCultB) in presence of Parathyroid hormone followed by 25 days in presence of insulin, HGF, hydrocortisone and FGF10.

### Example 4

### 2D- and 3D-lactocyte differentiation based on human-induced pluripotent stem cell (hiPSC) line 603

### (a) 3D-lactocyte differentiation based on human-induced pluripotent stem cell (hiPSC) line 603:

The human-induced pluripotent stem cell (hiPSC) line 603 was used for 3D-lactocyte differentiation. The human-induced pluripotent stem cell (hiPSC) line 603was purchased from Fujifilm Cellular Dynamics, Inc (FCDI).
(i) For the 3D differentiation protocol (according to the invention), EBs (spheroids) were formed by incubating single cells of hiPSC in E8 medium with 10uM rock inhibitor at 37°C, 5% CO2 in rotation at 95 rpm overnight.

Second day, medium was replaced with E8 (day -2-day 0).

Next day, medium was replaced with Mammo1 medium (MammoCult - medium with proliferation supplements, heparin (4µg/mL), and hydrocortisone (0.48µg/mL) with penicillin/streptomycin) for 10 days (day 0-day 10). Medium was changed every second day.
(ii) The differentiation was followed by 5 days in Mammo2 medium (EpiCultB + supplements, pTHrP 100ng/ml plus penicillin/streptomycin). Culture medium was changed every 3 days (day 10-day 15).
(iii) In order to induce branching epithelial structure,alveolar differentiation and mammary cell specification, mEBs (spheroids/mammospheres) were fed with Mammo3 medium (complete EpiCultB, hydrocortisone (1 µg/ml), insulin (10 µg/ml), FGF10 (50 ng/ml), HGF (50 ng/ml) and penicillin/streptomycin) for 20 days. Medium was changed every 3 days (day 15-day 35).
(iv) Finally, to induce the milk bioactive production (3D), we used the Mammo4 medium (complete EpiCultB, 10% FBS, prolactin (10 µg/ml), hydrocortisone (1 µg/ml), insulin (10 µg/ml), progesterone, β-estradiol and penicillin/streptomycin for 7 days and medium was changed every 3 days (day 35-day 42). During all the differentiation procedure, spheroids were maintained in the suspension culture (rotating at 95 rpm). The differentiation procedure ended at day 42. Results are displayed in Figure 4.

### (b) 2D-lactocyte differentiation based on human-induced pluripotent stem cell (hiPSC) line 603

The human-induced pluripotent stem cell (hiPSC) line 603 was used also for 2D-lactocyte differentiation. The human-induced pluripotent stem cell (hiPSC) line 603was purchased from Fujifilm Cellular Dynamics, Inc (FCDI).

For the 2D-differentiation protocol (used for comparison), we used the Lacto medium during all the differentiation stages (RPMI 1640, 20% FBS, 1mM glutamine, 4 µg/ml insulin, 20 ng/ml EGF, 0.5µg/ml hydrocortisone with penicillin/streptomycin). Cells were incubated at 37°C, 5% CO2. Medium was replaced every second day. Results are displayed in Figure 5.

### (c) Results

The different differentiation stages during lactocyte derivation were captured using quantitative RT-PCR (Figure 4, 3D-differentiation, Figure 5, 2D-differentiation). In both 2D- and 3D-settings, NaNog expression as a marker for pluripotency is decreased while cells are passing towards the maturation and differentiation. The neuroectodermal and endodermal markers, TUBB3 (Tubulin Beta 3 Class III) and Forkhead box protein A2 (FOXA2) were not expressed significantly in 3D-format and TUBB3 elevation is only captured in 2D-setting. This demonstrates that hiPSCs are patterned towards the non-neural ectodermal lineage, thus enriching mammary progenitors in 3D-format. We investigated the expression pattern of commonly used basal cell/myoepithelial markers, such as p63 (a p53-homologous nuclear protein) and cytokeratin 14 (KRT-14). Both markers are detectable significantly in both systems. Additionally, the epithelial cell adhesion molecule (EpCAM) and cytokeratin 8 (KRT8) were tracked only in the 3D-system and KRT8 was only partially expressed in the2D-format. Consequently, 3D-platfrom in an organotypic setting expressed common breast tissue, luminal, and basal markers. Such mammary like organoids express human breast specific proteins including CSN2 (casein beta), milk protein peptides, and hormone receptors. The luminal cells specifically express EpCAM, MUC1, CD49F, GATA3, CK8, and CK18 while basal cells will specifically express CK14, α-smooth muscle actin and P63. Eventually EpCAM and CD49F double positive cells can be detected at an earlier progenitor stage between D10 and D35. Interestingly, CSN2 expression is only captured at the last time point (D42) of the 3D-organotypic system and not in the 2D-directed differentiation platform.

Analysis of the mammary like organoids secretome showed secretion of human milk specific bioactives including oligosaccharides (including lactose and some HMOs), lipids (including 4 fatty acids), proteins (7 detected including caseins), and miRNA (75 detected, including 11 typically detected in HBM) as below described.

Primary cell supernatant was analyzed for presence of lactose or human milk oligosaccharides following the procedure described in "Austin and Benet, Quantitative determination of non-lactose milk oligosaccharides, Analytica Chimica Acta 2018, 1010, 86-96" with minor modification. The samples were analysed with UHPLC and detected lactose or human milk oligosaccharides (HMOs) were quantified against a calibration curve of lactose and a mix of 7 HMOs (2'FL, 3FL, DFL, LNT, LNnT, 3'SL and 6'SL). The method had an estimated limit of 0.1mg/L. In the primary cell supernatants, Lactose (0.22 mg/l) and 6'SL (0.32 mg/l) were detected at day 42.

Fatty acids were analysed in media and cell supernatants by gas chromatography coupled with flame ionization detector. Briefly, the supernatants obtained at day 42 is analysed to investigate the presence of fatty acids contained in several lipid classes. A 7890A gas-chromatograph with a 7693 autosampler with preparative station module equipped with a fused-silica CP-Sil 88 capillary column (100% cyanopropylpolysiloxane; 100 m, 0.25 mm id, 0.25 mm film thickness is used with a split injector (1:25 ratio) heated at 250°C and a flame-ionization detector operated at 300°C. Preparation of FAMEs (fatty acids methyl esters) is performed by direct transesterification of sample with methanolic chloridric acid. Separation of FAMEs is performed using capillary gas chromatography-FID (GC). Identification of FAMEs is done by retention time (RT) and comparison with an external standard. Quantification of fatty acids is done by calculation using methyl C11:0 as internal standard. Transesterification performance of the method is controlled with TAG C13:0 as second internal standard. After addition of internal standards, the solution was mixed with 2 mL of methanol, 2 mL of Methanol/HCl (3N) and 1 mL of hexane. After heating at 100°C/60min, the sample is cooled down to room temperature (about 15 min) and the reaction is stopped by adding 2mL of water. After centrifugation the organic phase is directly injected into the GC.

Fatty acid results from protocol of Example 4a at time day 42 are reported in table 2 (differences observed between media and supernatant).

The table 2 below lists the expressed fatty acids in cell supernatant sample.

| **Fatty acid** | **Detected amount in cell supernatant (mg/100 mL)** |
|---|---|
| C-4:0 | 2.53 |
| C-8:0 | 0.49 |
| C-10:0 | 0.38 |
| C-14:0 | 0.44 |
| C-15:0 | 0.41 |
| C-16:0 | 1.85 |
| C-16:1n7 | 0.08 |
| C-17:0 | 0.09 |
| C-18:0 | 0.97 |
| C-18:1 n9 | 18.82 |
| C-18:1 | 0.28 |
| C-18:2 n6 | 2.16 |
| C-20:0 | 0.13 |
| C-20:1 n9 | 0.11 |
| C-18:3 n3 | 0.08 |
| C-22:0 | 0.29 |
| Other fatty acids | 1.38 |

Proteins in the cell supernatant were analysed using SDS-PAGE profiling and then band isolation for identity confirmation by LC-MSMS. For SDS-PAGE analysis, the total volume of the prepared sample was loaded on the gel. A human milk sample was added for comparison as control. Selected gel regions (bands) were cut to look for human proteins by LC-MSMS. Eventually, bands were submitted to in-gel trypsin digestion and analyzed by LC-MSMS. LC-MSMS data were analyzed with Peaks Studio and matched against the UniProt database for human proteins.

The table 3 below lists the best candidates for all the excised bands.

| **Name of expressed proteins in the cell supernatant** |
|---|
| Lactoferrin |
| Albumin |
| Prolactin |
| Alpha S1-casein |
| Hemoglobin subunit beta |
| Hemoglobin subunit alpha |
| α-lactalbumin |
| Alpha-2-macroglobulin |
| β-casein |
| bile salt-activated lipase |
| K-casein |
| lactadherin |
| CD14 |
| fatty acid synthase |
| IgA |
| plgR |
| Serum albumin |
| Xanthine dehydrogenase |

Exosome isolation and miRNA profiling was performed using ExoQuick polymer nets. ExoQuick polymer works to precipitate exosomes by forming a network and collects all exosomes of a certain size. Once the ExoQuick mesh is formed, a simple, low-speed centrifugation easily precipitates the exosomes as a pellet. The exosomes are intact, ready for protein or RNA analysis and are bioactive for functional studies. Precipitation buffer was added in a ration 0.25X to the sample then vortex. The mix was incubated overnight at 4°c. After incubation, samples were centrifuged 30 min at 1,500xg. The exosome pellet was re-suspended by vertexing in initial volume with Buffer XE (QIAGEN) for QC or Lysis Buffer from HTG EdgeSeq miRNA Whole Transcriptome Assay for miRNA profiling. In order to assess the extracellular vesicles (EVs) isolation, the supernatant was first centrifuged at 3000g for 15 min to remove cell pellet and debris. Then 100 microliters of media was used for an overnight precipitation at 4°c with ExoQuick buffer (ratio 0.25X). EV precipitates were recovered by centrifugation for 30 min at 1500g. Two precipitations were performed for each sample, one EV precipitation was resuspended in Buffer XE (QIAGEN) for potential further analysis, and a second one in only 50 ul HTG Lysis buffer in order to concentrate by 10-fold before miRNA profiling with HTG.

For miRNA profiling, samples were used directly in the first step of lysis. Thus, Whole sample was used directly and was lysed with Plasma lysis buffer in a ratio1:1. Next, proteinase K (1/10) was added and the samples were incubated 3h at 50°c at 600rpm on Thermomixer. EVs were resuspended in Lysis buffer and lysed in the same conditions, with an incubation step at 95°c for 10min added before the lysis incubation. 26 µl of lysate was process with 70 µl of oil on the HTG processor following the HTG EdgeSeq miRNA Whole Transcriptome Assay V2 procedure. For indexing and amplification libraries, samples were tagged with Illumina adaptors and indexes by PCR with OneTaq^{®} Hot Start 2X Master Mix GC Buffer (95°C - 4 min; 16 cycles: 95°C-15 sec, 56°C-45 sec, 68°C- 45 sec; 68°C10 min; Hold at 4°C) and AMPure cleaned (ratio 2.5) on a robotic liquid handler SciClone NGS WorkStation (Perkin Elmer). Pools were obtained with our custom pooling program on Hamilton robot. The samples were pooled based on GX touch Chip HS quantification. The pools were purified manually a second time with AMPure Bead (ratio 1.8) to remove potential remaining traces of primer-dimer and quantified with Qubit to adjust the final concentration to 2 nM. And as a last step, for MiSeq sequencing, pools were loaded on MiSeq at 20pM with a 5% PhiX spike and sequenced for 50 base Single read on MiSeq with 150V3 kit.

Briefly, 974 miRNAs detected in the in the cell supernatant which more than 75 of them are highly expressed miRNAs in the milk samples.

The table 4 below lists the top ten highly expressed miRNAs.

| **miRNA name** | **log2 counts** | **CV** |
|---|---|---|
| miR-21-5p | 9.76 | 0.01 |
| miR-181a-5p | 9.07 | 0.03 |
| miR-30d-5p | 8.63 | 0.01 |
| miR-30b-5p | 8.63 | 0.01 |
| miR-22-3p | 8.49 | 0.01 |
| miR-146b-3p | 8.40 | 0.01 |
| miR-30c-5p | 8.12 | 0.04 |
| miR-30a-5p | 7.63 | 0.02 |
| miR-30e-5p | 7.26 | 0.01 |
| miR-148b-3p | 6.77 | 0.04 |

Our findings provide a novel iPSC-based 3D-organotypic model for studying the regulation and development of normal mammary cell fate and function as well as breast milk bioactives production.

### Example 5

In order to generate milk bioactives that closely resemble those found in human breast milk, different methods using bone morphogenetic protein 4 (BMP4) and retinoic acid (RA) in 42 days and 31 days as a shortened time-course protocol were established in a 3D-platform using iPSCs derived mammary gland organoids as a biomimetic model of the human mammary gland (Figure 6).

3D-iPSC cells in 42 days protocol and shortened time-course protocol (31 days -* *describes shortened-modified protocols (31 days) including BMP4 and RA combinations*) using 20 ng/mL of BMP4 and 1 µM of RA in single or combination format compared to the control condition can induce the expression of mammary gland progenitor markers such as EpCAM (CD326), CD49f, MUC1 (CD227) and GATA3 using flow cytometry quantification or maintain their expression profile during the differentiation time-course and/or pre-induction stages (Figure 7a-c) and post-induction period (Figure 7d-f).

We assessed the expression of different mammary epithelium markers in lactocytes derived from iPSCs in normal length and shortened protocols (Figure 8). This shortened protocol induces RELA as a specific gene for determining the lineage of the mammary epithelial cells during iPSC differentiation (Figure 8a). In addition to the mature luminal markers such as GATA3, specific lactocyte markers such as EpCAM, KRT8/18 are also induced in the shortened protocol (Figure 8b-e). In addition, when we switch to the 31-day protocol, the ESRRA level is inducted (Figure 8f). As well, the shortened differentiation progress is associated with elevated levels of progenitor markers for mammary glands such as CD24 and ITGA6 (CD49f) (Figure 8g-h).

Human osteopontin peptides - GDSVVYGLR and -YPDAVATWLNPDPSQK were specifically detected in the cell culture supernatant by LC-MS/MS (Figure 9).

### Example 6

A more systematic analysis of one specific human breast milk bioactive component, exosomes, as produced by Step A revealed very close profiles of composition with exosomes from human breast milk positive control.

Human iPSC were aggregated in 3D-culture using ultra-low binding 6-well plates with continuous rotation shacking to form embryoid bodies, and then subjected to a multi-steps differentiation protocol (41 days) to generate functional mammary gland organoids. The last 7 days of the differentiation protocol (from day 35 to day 41), the mammary gland organoids were subjected to a specific culture media supplemented with lactogenic hormones and growth factors to induce the secretion of milk-specific bioactive compounds. This media was renewed every other day (Day 35, Day 37, and Day 39).

Conditioned media was collected at Day 39 and Day 41 from step A, and pooled (which corresponds to the media that was in contact with the cells from Day 37 to Day 41). EVs were purified from the conditioned media and from skim human milk (commercially available at Lee Biosolutions, 991-01-P, collected from at least 5 lactating women after week 4 of lactation) by ultracentrifugation.

Analysis of the EV-associated protein profile by liquid chromatography - mass spectrometry (LC-MS) showed an overlap of approximately 30-50% when comparing breast milk control EVs and cell-based derived EVs (Figure 10a). Figure 10b shows that most of the proteins highly expressed in breast milk exosomes are also expressed in the cell-based exosomes including lactotransferrin (LTF), annexin A2 (ANXA2) and lactadherin (MFGE8), but also Muc1, CD9, CD81, and CD63. Interestingly some proteins such as C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine—tRNA ligase 1, cytoplasmic (TARS1), and Cytosolic non-specific dipeptidase (CNDP2), are not detected in cell-based EVs but only in breast milk EV positive control.

Analysis of the miRNA profile of EVs sequenced with HTG Molecular Whole Transcriptome Assay showed strong 95-99% overlap between breast milk and cell-based exosomes (Figure 11a), including detection of already validated miRNA such as miR-148a-3p, miR-22-3p, miR-125b-5b and miR-6126 (Figure 11b).

Analysis of the lipid fractions with the Lipometrix method (LC-MS/MS untargeted lipidomic analysis) in EVs purified from both conditioned media and human milk revealed similar overall profiles of lipid classes including cholesterol esters, ceramides, triacylglycerides, diacylglycerides, total phospholipids, alkanyl phospholipids, and alkenyl phospholipids (Figure 12). Same observation when analyzing subclasses profiles as phospholipid profile.

Flow cytometry analysis of the isolated EVs either from conditioned media (StepA) or from breast milk control showed a similar size ranging from 65 nm to 75nm.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. An isolated human breast milk exosome, wherein the exosome does not comprise one or more proteins selected from C4a anaphylatoxin (CFA), Hypoxia up-regulated protein 1 (HYOU1), Apolipoprotein A-IV (APOA4), Procollagen-lysine,2-oxoglutarate 5-dioxygenase 1 (PLOD1), Threonine—tRNA ligase 1, cytoplasmic (TARS1), and Cytosolic non-specific dipeptidase (CNDP2).

2. The exosome of claim 1, wherein the exosome comprises lactotransferrin (LTF), annexin A2 (ANXA2) and lactadherin (MFGE8), MUC1 and tetraspanins CD9, CD81 and CD63.

3. The exosome of claim 1 or 2, wherein the exosome comprises one or more miRNAs selected from miR-148a-3p, miR-22-3p, miR-125b-5b and miR-6126.

4. The exosome of any preceding claim, wherein the exosome comprises one or more lipids selected from cholesterol esters, ceramides, triacylglycerides, diacylglycerides, lyso-phospholipids, total phospholipids, alkanyl phospholipids, and alkenyl phospholipids.

5. The exosome of any preceding claim, wherein the exosome comprises one or more phospholipids selected from sphingomyelins, phosphatidylcholine, phosphatidylethanolamine, phosphatidylinositol, and phosphatidylserine.

6. The exosome of any preceding claim, wherein the exosome has a diameter of 50 to 100nm.

7. The exosome of claim 6, wherein the exosome has a diameter size of 65 to 75nm.

8. The exosome of any preceding claim, wherein the exosome has the same function as exosomes derived from natural human breast milk.

9. The exosome of any preceding claim, wherein the exosome has the same structure as exosomes derived from natural human breast milk.

10. A population of exosomes, wherein the population comprises exosomes of any preceding claim.

11. A composition comprising exosomes or a population of exosomes according to any preceding claim.

12. An *in vitro* method for producing an exosome or population or exosomes according to any one of claims 1 to 11, the method comprising:
A) Generating lactocyte mammary-like gland organoids derived from human induced pluripotent stem cells (hiPSC),
B) Secreting a human milk like product from said lactocytes, and
C) Purifying the exosomes from the human milk like product to remove impurities, optionally by chromatography or filtration or ultracentrifugation, in order to isolate the exosomes,
wherein step A) comprises culturing the hiPSCs in a culture medium comprising BMP4 and/or RA.

13. The method of claim 12, wherein step A) is between a 30-day and 45-day process.

14. The method of claim 12 or 13, wherein Step A) is conducted in 3D suspension culture conditions.

15. The method of claims 12 to 14, wherein step C) further comprises formulating the isolated exosomes into a powder form, optionally by spray drying or freeze drying, or liquid form.

16. The method of claims 12 to 15, wherein step C) further comprises sterilising the isolated exosomes.

17. The method of claims 12 to 16, wherein step C) further comprises storing the isolated exosomes at below freezing, optionally at -80°C.

18. The method of claims 12 to 17, further comprising formulating the isolated exosomes with supplementary nutritional ingredients.

19. The method of claims 12 to 18, wherein the isolated exosomes are dispensed into a container for consumption.

20. A human breast milk exosome product which is obtainable according to the method of any one of claims 12 to 19.

21. A method of producing a human milk fortifier, comprising conducting the method of any one of claims 12 to 19.
